(19) Europäisches Patentamt
European Patent Office
Office européen des brevets

(11) Publication number: **0 132 367**
**B1**

# (12) EUROPEAN PATENT SPECIFICATION

(45) Date of publication of patent specification: **08.06.88**

(21) Application number: **84304858.8**

(22) Date of filing: **17.07.84**

(51) Int. Cl.[4]: **C 07 C 59/68,** C 07 C 59/66, C 07 C 57/58, C 07 C 59/52, C 07 C 59/88, C 07 C 59/90, C 07 C 149/36, C 07 C 149/40, C 07 C 149/41, C 07 C 149/437, C 07 D 257/06

## (54) Leukotriene antagonists.

(30) Priority: **18.07.83 US 514394**

(43) Date of publication of application:
**30.01.85 Bulletin 85/05**

(45) Publication of the grant of the patent:
**08.06.88 Bulletin 88/23**

(84) Designated Contracting States:
**AT BE CH DE FR GB IT LI LU NL SE**

(56) References cited:
**DE-A-1 124 296**
**FR-A-2 509 725**
**FR-M- 4 722**
**GB-A-1 111 361**
**GB-A-1 591 063**

(73) Proprietor: **ELI LILLY AND COMPANY**
**307, East McCarty Street**
**Indianapolis Indiana 46285 (US)**

(72) Inventor: **Herron, David Kent**
**5945 Andover Road**
**Indianapolis Indiana 46220 (US)**

(74) Representative: **Hudson, Christopher Mark et al**
**Erl Wood Manor**
**Windlesham Surrey GU20 6PH (GB)**

**EP 0 132 367 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Research in the area of allergic reactions of the lung has provided evidence that archidonic acid derivatives formed by the action of lipoxygenases are related to various disease states. Some of these arachidonic acid metabolites have been classified as members of a family of eicosatetraenoic acids termed leukotrienes. Three of these substances are currently thought to be major components of what has been previously called slow reacting substance of anaphylaxis (SRS—A).

This invention provides novel chemical agents which are selective leukotriene antagonists that can be used therapeutically in the treatment of allergic disorders such as asthma, where leukotrienes are thought to be causal mediators.

More specifically, the invention provides compounds of the Formula I

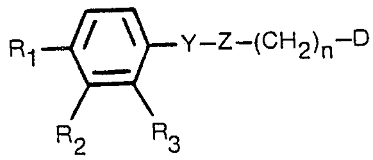

I

and salts thereof, wherein:

$R_1$ is $R_5-\overset{O}{\overset{\|}{C}}-$ or halo;

$R_2$ is halo or hydroxy;

$R_3$ is $C_1$—$C_{12}$ alkyl, hydroxy-substituted $C_1$—$C_{12}$ alkyl, or $C_2$—$C_6$ alkenyl;

Y is —O—, $-\overset{(O)_p}{\overset{\|}{S}}-$, or $-CR_6R_7-$;

Z is —O—, $-\overset{(O)_p}{\overset{\|}{S}}-$, or $-CR_8R_9-$,

or when taken together,

—Y—Z is —CH=CH—;

n is 1—10;

D is CN, SCN or $QR_4$

Q is —O—, —NR—, $-\overset{(O)_p}{\overset{\|}{S}}-$ or a bond;

$R_4$ is $-COR_{10}$, hydroxy, $-NR_{11}R_{12}$, —SC(=NH)$NH_2$, or

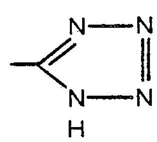

where

R is hydrogen or $C_1$—$C_3$ alkyl;

$R_5$ is hydrogen, $C_1$—$C_6$ alkyl, $C_3$—$C_8$ cycloalkyl, or phenyl optionally substituted with halo, $C_1$—$C_4$ alkyl, or $C_1$—$C_4$ akoxy;

each of $R_6$ and $R_7$ is independently hydrogen, $C_1$—$C_{10}$ alkyl, phenyl, or benzyl;

$R_8$ and $R_9$ are hydrogen;

$R_{10}$ is hydroxy, $C_1$—$C_4$ alkoxy, —NHOH, or $-NR_{11}R_{12}$;

each of $R_{11}$ and $R_{12}$ is independently hydrogen, $C_1$—$C_3$ alkyl, or when taken together with the nitrogen atom form a morpholine or N-methyl piperazine ring; and

p is 0, 1, or 2;

with the provisions that:

a) when $R_1$ is

$$R_5-\overset{\overset{\large O}{\|}}{C}-$$

$R_2$ may not be hydroxy;

b) when one of Y and Z is

$$-O- \text{ or } -\overset{\overset{(O)_p}{\|}}{S}-),$$

the other of Y and Z may not be

$$-O- \text{ or } -\overset{\overset{(O)_p}{\|}}{S}-; \text{ and}$$

c) when $R_4$ is $COR_{10}$, hydroxy, $-NR_{11}R_{12}$, or $-SC(=NH)NH_2$, Q may only be a bond.

Compounds of formula (I) wherein D is CN or SCN are useful as intermediates in preparation of pharmaceutically useful compounds of formula (I) wherein D is $QR_4$.

Compounds in which D is $QR_4$ and each of $R_8$ and $R_9$ is independently hydrogen, $C_1$—$C_{10}$ alkyl, phenyl, or benzyl, are provided for the manufacture of a medicament for treating or preventing any condition characterized by excessive release of leukotrienes, or for treating or preventing immediate hypersensitivity reaction.

A preferred group of compounds are the compounds of Formula I wherein:

(a) $R_1$ is halo, especially chloro,

(b) $R_2$ is halo, especially chloro,

(c) $R_3$ is $C_1$—$C_6$ alkyl, especially propyl,

(d) Y is O—,

(e) Z is $-CR_8R_9-$

(f) Q is —O— a bond, or —S— (p is 0),

(g) $R_4$ is 5-tetrazolyl or —COOH, and

(h) n is 1—4.

An especially preferred group of compounds are those of the formula Ia

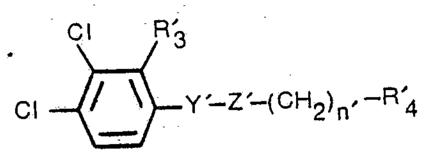

Ia

and pharmaceutically acceptable salts thereof wherein:

$R_3'$ is $C_1$—$C_6$ alkyl, especially propyl;

each of Y′ and Z′ is independently

$$-O-, -\overset{\overset{(O)_p}{\|}}{S}-, \text{ or } -CH_2-,$$

except that when one of Y′ and Z′ is

$$-O- \text{ or } -\overset{\overset{(O)_p}{\|}}{S}-,$$

the other of Y′ and Z′ may not be

$$-O- \text{ or } -\overset{\overset{(O)_p}{\|}}{S}-;$$

n′ is 2 or 3; and

$R_4'$ is —COOH, 5-tetrazolyl, or 5-thiotetrazolyl.

The term "$C_1$—$C_{12}$ alkyl" refers to the straight and branched aliphatic radicals of 1 to 12 carbon atoms such as methyl, ethyl, propyl, isopropyl, butyl, isobutyl, sec-butyl, tert-butyl, amyl, isoamyl, sec-amyl, sec-isoamyl (1,2-dimethylpropyl), tert-amyl (1,1-dimethylpropyl), hexyl, isohexyl (4-methylpentyl), sec-hexyl (1-methylpentyl), 2-methylpentyl, 3-methylpentyl, 1,1-dimethylbutyl, 2,2-dimethylbutyl, 3,3-dimethylbutyl, 1,2-dimethylbutyl, 1,3-dimethylbutyl, 1,2,2-trimethylpropyl, 1,1,2-trimethylpropyl, heptyl, isoheptyl (5-methylhexyl), sec-heptyl (1-methylhexyl), 2,2-dimethylpentyl, 3,3-dimethylpentyl, 4,4-dimethylpentyl, 1,2-dimethylpentyl, 1,3-dimethylpentyl, 1,4-dimethylpentyl, 1,2,3-trimethylbutyl, 1,1,2-trimethylbutyl, 1,1,3-trimethylbutyl, octyl, isooctyl (6-methylheptyl), sec-octyl (1-methylheptyl), tert-octyl (1,1,3,3-tetramethylbutyl), nonyl, 1-, 2-, 3-, 4-, 5-, 6-, or 7-methyloctyl, 1-, 2-, 3-, 4-, or 5-ethylheptyl, 1-, 2-, or 3-propylhexyl, decyl, 1-, 2-, 3-, 4-, 5-, 6-, 7-, or 8-methylnonyl, 1-, 2-, 3-, 4-, 5-, or 6-ethyloctyl, 1-, 2-, 3-, or 4-propylheptyl, undecyl, dodecyl, and the like. The term "$C_1$—$C_{12}$ alkyl" includes within its definition the terms "$C_1$—$C_3$

alkyl", "$C_1$—$C_4$ alkyl", and "$C_1$—$C_6$ alkyl."

The term "$C_3$—$C_8$ cycloalkyl" refers to the saturated alicyclic rings of three to eight carbon atoms such as cyclopropyl; methylcyclopropyl, cyclobutyl, cyclopentyl, cyclohexyl, cyclooctyl, and the like.

The term "$C_2$—$C_6$ alkenyl" refers to straight and branched radicals of two to six carbon atoms such as ethenyl, allyl, isopropenyl, butenyl, isobutenyl, 3-methyl-2-butenyl, n-hexenyl, and the like, and includes the term "$C_3$—$C_6$ alkenyl."

The term "halo" refers to fluoro, chloro, bromo, and iodo. The term "$C_1$—$C_4$ alkoxy" refers to straight and branched alkoxy radicals of up to four carbon atoms such as methoxy, ethoxy, n-propoxy, isopropoxy, n-butoxy, tert-butoxy, and the like.

The pharmaceutically acceptable base addition salts of this invention include salts derived from inorganic bases, such as ammonium and alkali and alkaline earth metal hydroxides, carbonates, bicarbonates, and the like, as well as salts derived from non-toxic basic organic amines, such as aliphatic and aromatic amines, aliphatic diamines, hydroxy alkylamines, and the like. Such bases useful in preparing the salts of this invention thus include ammonium hydroxide, potassium carbonate, sodium bicarbonate, calcium hydroxide, methyl amine, diethyl amine, ethylene diamine, cyclohexylamine, ethanolamine, and the like. The potassium and sodium salt forms are particularly preferred.

In addition, when the compounds of formula I are amine derivatives (*e.g.*, $R_4$ is $-NR_{11}R_{12}$ or $-SC(=NH)NH_2$), the compounds may also exist as the corresponding acid addition salts. The pharmaceutically acceptable acid addition salts of this invention therefore also include salts derived from inorganic acids such as hydrochloric acid, nitric acid, phosphoric acid, sulfuric acid, hydrobromic acid, hydriodic acid, phosphorous acid and the like, as well as salts derived from nontoxic organic acids such as aliphatic mono and dicarboxylic acids, phenyl-substituted alkanoic acids, hydroxy alkanoic and alkandioic acids, aromatic acids, aliphatic and arommatic sulfonic acids, etc. Such pharmaceutically acceptable salts thus include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, mandelate, butyne-1,4-dioate, hexyne-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, β-hydroxybutyrate, glycollate, malate, tartrate, methanesulfonate, propanesulfonate, naphthalene-1-sulfonate, naphthalene-2-sulfonate, and the like salts. Salts from inorganic acids are preferred, especially the hydrochloride or hydrobromide salts.

It is recognized that if $R_6$ is different from $R_7$, or $R_8$ is different from $R_9$, various stereoisomers will exist. This invention is not limited to any particular stereoisomer but includes all possible individual isomers and racemates of the compounds of Formula I. Similarly, when —Y—Z— is —CH=CH—, both the individual *cis* and *trans* isomers and their mixture are included as part of this invention.

Some of the compounds of this invention may be prepared by the reaction of a compound of the formula

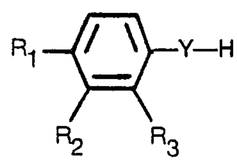

(II)

wherein Y is —O— or —S—, with a compound of the formula

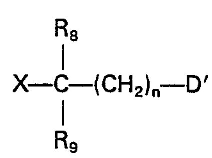

III

wherein X is a suitable leaving group, such as halo, preferably chloro, and D is $-Q-R_4$, a precursor of $-Q-R_4$, halo, cyano, thiocyano, or a protected acid ester such as a benzhydryl ester. This procedure is useful in preparing the compounds of this invention designated by Formula I'

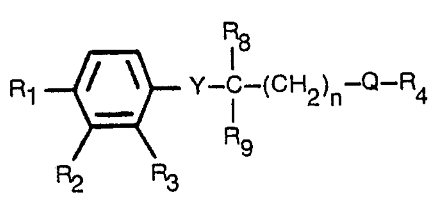

I'

either directly (when D' is $Q-R_4$ or indirectly from intermediates IV

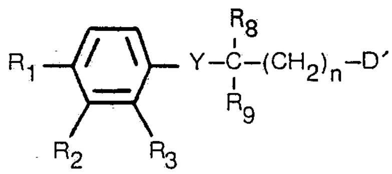

IV

wherein D′ is a precursor to —Q—$R_4$, halo, cyano, thiocyano, or a protected acid ester.

The reaction between compounds II and III is usually performed in equimolar amounts although ratios other than equimolar amounts are completely operative. Preferably the molar ratio of compound II to compound III is in the range of 1:1 to 1:4. The reaction is best carried out in a nonreactive solvent such as ketones, especially acetone or methyl ethyl ketone, and in the presence of a base, preferably sodium hydride or an alkali metal hydroxide or carbonate, preferably potassium carbonate. Preferably from 1 to 5 equivalents of base are used for mole of compound II. Especially when X is chloro, a catalyst such as potassium or sodium iodide may be added to increase the reaction rate. The reaction may be carried out at temperatures of about ambient temperature up to the boiling point of the reaction mixture, the latter being preferred.

In the case where D′ is cyano, the resulting intermediate IV may be converted to further compounds of this invention by the following methods. Compounds of Formula I′ wherein $R_4$ is —COOH may be obtained by hydrolysis of the intermediate cyano derivative. This is generally accomplished by heating the cyano derivative in aqueous alcohols in the presence of a base such as sodium hydroxide. Alternatively, the carboxylic acid derivatives (I′, $R_4$ is —COOH) may be prepared by the hydrolysis of the corresponding ester derivatives. This may be accomplished by an aqueous hydrolysis as described above or, especially in the case of a diphenylmethyl(benzhydryl)ester, using methods known in the art such as treating with formic acid and triethylsilane followed by an aqueous workup, acidic hydrolysis, treatment with trifluoroacetic acid in anisole, or catalytic hydrogenation. The required benzhydryl ester starting materials (III, D′ is a benzhydryl ester) may be prepared from the corresponding carboxylic acids (III, D′ is —COOH) in the usual ways, such as treatment with diphenyldiazomethane in methylene chloride or heating with benzhydrol and a mineral acid in a solvent such as toluene with the azeotropic removal of water. The compounds of Formula I′ wherein $R_4$ is —COO($C_1$—$C_4$ alkyl) may be prepared by conventional methods of esterification from the respective acid derivatives or are prepared directly by the methods described below. Salts may be prepared by treating the corresponding acids ($R_4$ is —COOH) with an appropriate base in the normal manner. Amide derivatives ($R_4$ is —$CONR_{11}R_{12}$ or —CONHOH) may be prepared by direct aminolysis of the corresponding ester, or from the corresponding carboxylic acid using conventional means such as conversion to the acid chloride followed by reaction of the acid chloride with an appropriate amine or treatment with an agent such as 1,1′-carbonyldiimidazole in the presence of an appropriate amine. In either case, the ester or acid is reacted with the appropriate amine V

$$HNR_{11}R_{12} \qquad V$$

wherein $R_{11}$ and $R_{12}$ are as described hereinabove, or hydroxylamine, the latter giving the hydroxamic acid derivative.

The compounds of Formula I′ wherein $R_4$ is 5-tetrazolyl (Q is a bond) are prepared by treating the cyano intermediate with an alkali metal azide such as sodium azide, ammonium chloride, and (optionally) lithium chloride in a non-reactive high-boiling solvent such as N,N-dimethylformamide, preferably at temperatures from 60°C to the reflux temperature of the reaction mixture. Alternatively, tetramethylguanidinium azide may be used in place of the alkali metal azide, ammonium chloride, and lithium chloride. The thiotetrazole compounds of Formula I′ (Q is —S—) are prepared from the thiocyano intermediates in a similar manner or may be prepared from a halo intermediate (IV, D′ is halo) on treatment with 5-mercaptotetrazole.

When employing intermediate III wherein D′ is halo, those skilled in the art will recognize that when the substituents $R_8$ and $R_9$ are both hydrogen, affording a symmetrically-substituted dihaloalkane III, X and D′ may be the same or different leaving groups since the reaction with compound II will give the same product IV regardless which "end" of the molecule reacts. However, when alkane III is non-symmetrically substituted, those skilled in the art will recognize that X should be a better leaving group than D′ (halo) in order for the desired product IV to be formed. If D′ is the better leaving group in compound III, III can first be converted to a different intermediate compound III (*e.g.*, reaction of III (D′ is halo) with an alkali metal cyanide to give III (where D′ is —CN)) which can then be reacted with compound II as previously described.

The compounds of Formula IV wherein D′ is halo may be transformed into the compounds of this invention in the following manner. When compounds of Formula IV (D′ is halo) are heated with an alkali metal cyanide, such as sodium cyanide, in the presence of a high boiling, nonreactive solvent, such as N,N-dimethylformamide, at elevated temperatures (50°C to the reflux temperature of the solvent), the intermediate cyano compound of Formula IV (D′ is cyano) is produced which may then be transformed into the acid, ester, or tetrazole derivatives as described previously. Similarly, the thiotetrazole compounds of this invention can be prepared by reacting a compound of Formula IV (D′ is halo) with an alkali metal thiocyanate in a similar manner to give the intermediate thiocyano compound of Formula IV (D′ is —SCN)

followed by transformation to the thiotetrazole in the usual manner. Alternatively, the thiotetrazole compounds may be prepared from IV (D' is halo) and 5-mercaptotetrazole in a similar manner as previously mentioned.

The compounds of Formula I' wherein $R_4$ is —OH may be prepared directly from the reaction of compound II and a haloalkanol (III, X is halo, D' is —OH) or may be prepared from the intermediate IV where D' is halo by aqueous hydrolysis. These compounds may be transformed into other compounds or intermediates of this invention (*e.g.*, where $R_4$ is —CN, etc.) by preparation of the mesylate derivative and displacing with a suitable nucleophile (such as cyanide ion).

The compounds of Formula I' wherein $R_4$ is $—NR_{11}R_{12}$ may be prepared by the reaction of the compounds of Formula IV where D' is halo with compounds of the Formula V. The reaction of compounds IV and V is generally carried out in the presence of a nonreactive, high-boiling solvent such as N,N-dimethylformamide, usually in the presence of a base, preferably an alkali metal carbonate or hydroxide, generally at elevated temperatures up to the boiling point of the solvent.

The isothiourea and thio-, amino-, and oxytetrazole compounds may be prepared from intermediate IV where D' is halo by reacting with thiourea and 5-mercapto-, 5-amino-, and 5-hydroxytetrazole, respectively. The reactions are performed by stirring the two reactants in a non-reactive solvent preferably at room to reflux temperature for about two to three days. In the thiourea reaction, ethanol is the preferred solvent and the product is usually isolated as the isothiuronium hydrohalide salt which is formed directly. In the tetrazole reactions, the preferred solvent is dimethylformamide and an acid scavenger, such as an alkali metal carbonate, is preferably included in the reaction.

Certain other compounds of this invention as defined by Formula I''

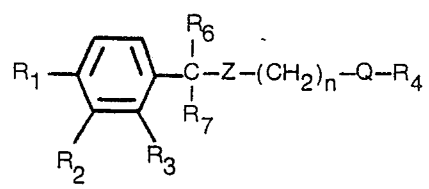

I''

are prepared in a similar manner as taught for the compounds of Formula I'. The compounds of Formula I'' are prepared directly or indirectly by treating a bromo-compound of the Formula VI

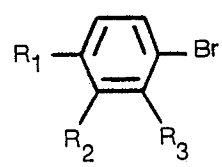

VI

with a strong base, such as lithium diisopropylamide, in an inert solvent, such as diethyl ether, at low temperatures, preferably −20 to 0°C., to prepare the lithium salt of VI which is then reacted with III'

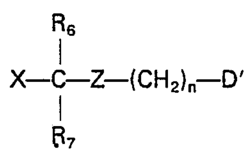

III'

to provide compounds I'' directly (when D' is $—Q—R_4$) or compounds VII.

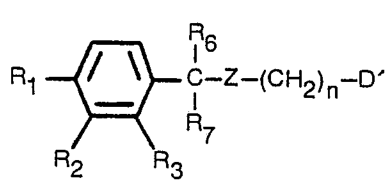

VII

Compounds VII can then be transformed into I'' by the same methods of transformation as previously described for converting compounds IV into I'.

Alternatively, when Z is —O— or —S—, certain compounds of Formula I'' may be prepared by reacting the appropriate benzyl derivative VIII

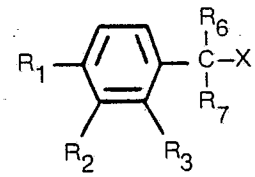

VIII

with a compound of the Formula IX

$$H—Z—(CH_2)_n—D'$$ IX

wherein Z is —O— or —S—, to give compounds I'' (Z is —O— or —S—) directly or indirectly through intermediate VII.

An additional preparation of certain compounds of Formula I'' (Z is —O— or —S—) involves the reaction of a benzyl compound XI

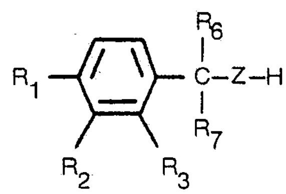

XI

wherein Z is —O— or —S—, with a compound of Formula XII

$$X—(CH_2)_n—D'$$ XII

to similarly give compounds I'' (Z is —O— or —S—) directly or indirectly through intermediates VII.

The alkene derivatives of this invention I'''

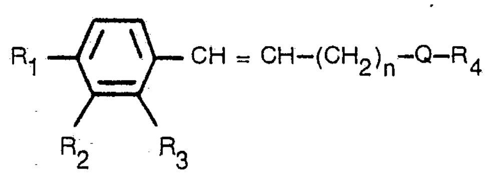

I'''

are prepared by reacting a Wittig reagent such as that represented by Formula XIII

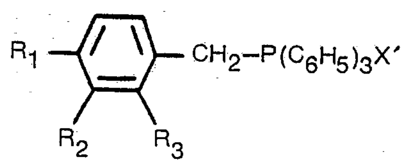

XIII

with an aldehyde of Formula XIV

$$O{=}CH(CH_2)_n—D'$$ XIV

wherein X' is chloro, bromo, or iodo, to give either compounds I''' directly (D' is —Q—$R_4$) or indirectly through intermediates XV. The transformations of intermediate XV

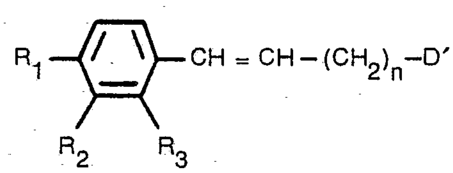

XV

to compounds I''' and the intraconversion of various compounds of Formula I''' are the same as previously described for compounds I' and I''. This sequence is limited, as those skilled in the art will appreciate, to those aldehydes XIV which may be prepared and are stable to the reaction conditions. For those substituents D which are unstable in the presence of aldehydes and/or Wittig conditions, the desired substituent may be introduced from an intermediate aldehyde after transformation into compound XV.

Additionally, the compounds of Formula I''' may be converted to the compounds of Formula I wherein Y and Z taken together are $—CH_2—CH_2—$ ($R_6$, $R_7$, $R_8$, and $R_9$ are all hydrogen) by the hydrogenation of I''' by any of many methods known in the art.

Compounds I''' and intermediates XV may also be produced from benzaldehyde XVI

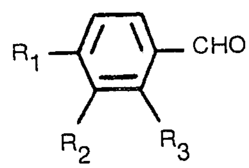

XVI

and Wittig reagent XVII

$$X'(C_6H_5)_3P—(CH_2)_{n+1}—D' \qquad XVII$$

in the same manner as described above for compounds XIII and XIV. This allows for the preparation of those compounds which could not be made due to the incompatability of the D' functionality and the aldehyde group in intermediate XIV.

The thio derivatives and intermediates of this invention (p is 0) may be transformed into the corresponding sulfoxide (p is 1) compounds upon treatment with a mild oxidizing agent, such as hydrogen peroxide in methanol or an alkali metal periodate in aqueous alcohol. The corresponding sulfones (p is 2) are prepared from the thio or sulfoxide compounds on treatment with a strong oxidizing agent such as hydrogen peroxide in acetic acid or m-chloroperbenzoic acid in methanol.

In addition, various compounds of Formula I can be prepared from other compounds, precursors, or intermediates of Formula I by standard methods such as hydrolysis, esterification, alkylation, oxidation, reduction, aminolysis, and the like, as are well known to those skilled in the art. In the prior discussion, the terms "precursor" and "precursor to $—Q—R_4$" mean those compounds, either related to the final compounds I or any intermediates or starting materials, which can be transformed into the desired functionality $—Q—R_4$. Those include the cyano intermediates and intermediates which may be transformed into the title products by any of the above mentioned methods known to those skilled in the art.

Other precursors to the products of this invention include phenyl compounds of Formula I wherein $R_1$, $R_2$ or $R_3$ are hydrogen. The desired substituents $R_1$, $R_2$ or $R_3$ can be introduced after the respective "halves" of the final compound have been coupled by methods previously described. These substituents can be introduced from the corresponding hydrogen substituted phenyl group by methods known in the art. For instance, the $R_1$ acyl substituents can be introduced following standard methods (*e.g.*, Friedel-Crafts reaction), halo groups of $R_1$ and/or $R_2$ can be introduced by halogenation of the phenyl ring, $R_3$ groups can be introduced by direct alkylation of the phenyl ring, etc. Although this functionalization of the phenyl ring can be performed in certain cases as the last step in the chemical sequence, those skilled in the art will recognize that such modifications will often be limited by the presence of interfering functionalities in the rest of the molecule and that it is preferred that such functionalizations be performed prior to coupling of the respective chemical "halves."

Intermediate compounds II, III, V, VI, VIII, IX, XI, XII, XIII, XIV, XVI, and XVII are either commercially available, known in the literature, or can be prepared according to methods known in the art.

Thus, one aspect of the invention is a process for preparing a compound of formula (I) which comprises

(a) reacting a compound of formula (II)

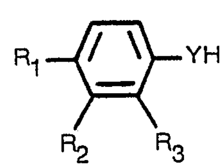

(II)

wherein $R_1$, $R_2$, and $R_3$ are as defined in formula (I), and Y is S or O, with a compound of formula (III)

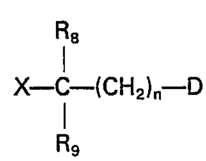

(III)

wherein $R_8$, $R_9$, and n are as defined in formula (I), X is a leaving group, and D is CN, SCN, or $QR_4$ as defined in formula (I) to provide a compound of formula (I) wherein Y is S or O; or

(b) reacting a compound of the formula

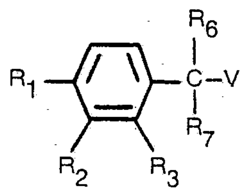

wherein $R_1$, $R_2$, $R_3$, $R_6$, and $R_7$ are as defined in formula (I) with a compound of the formula

$$W—(CH_2)_n—D$$

wherein n is as defined in formula (I), D is CN, SCN, or $QR_4$ as defined in formula (I), and one of V and W is a leaving group and the other is —ZH, where Z is O or S to provide a compound of formula (I) wherein Z is O or S; or

(c) reacting a compound of formula (XIII)

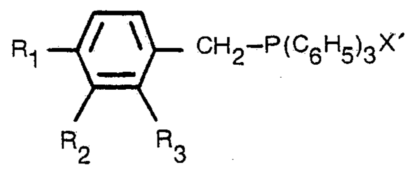

XIII

wherein $R_1$, $R_2$, and $R_3$ are as defined in formula (I) and X' is halo, with a compound of formula (XIV)

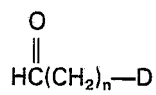

wherein n is as defined in formula (I) and D is CN, SCN, or $QR_4$ as defined in formula (I), to provide a compound of formula (I) wherein Y—Z is —CH=CH—; or

(d) reacting a compound of formula (XVI)

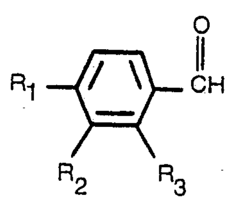

XVI

wherein $R_1$, $R_2$, and $R_3$ are as defined in formula (I), with a compound of the formula (XVII)

$$X'(C_6H_5)_3P—(CH_2)_{n+1}—D \qquad (XVII)$$

wherein n is as defined in formula (I), X' is halo, and D is CN, SCN, or $QR_4$ as defined in formula (I) to provide a compound of formula (I) wherein Y—Z is —CH=CH—; or

(e) reacting a compound of formula (I) wherein D is CN with an alkali metal azide and ammonium chloride, or with tetramethylguanidinium azide to provide a compound of formula (I) wherein D is 5-tetrazolyl; or

(f) reacting a compound of formula IV

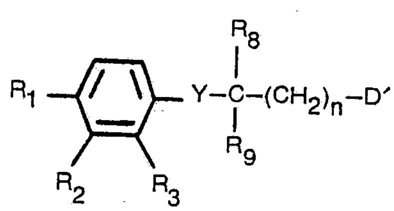

IV

wherein $R_1$, $R_2$, $R_3$, $R_9$, $R_8$, and n are as defined in formula (I) Y is O or S, and D' is halo, with an alkali metal cyanide or an alkali metal thiocyanate to provide a compound of formula (I) wherein D is CN or SCN; or

(g) reacting a compound of formula IV wherein D' is halo with 5-mercaptotetrazole to provide a compound of formula (I) wherein D is thiotetrazole; or

(h) oxidizing a compound of formula (I) wherein Y or Z is S or SO to provide the corresponding compound of formula (I) wherein Y or Z is SO or $SO_2$; and

(i) optionally salifying the resulting compound of formula (I).

Illustrative of the compounds of this invention are the following:

5-[4-(2-propyl-3,4-dichlorophenyl)butylthio]tetrazole,
N-methyl-8-(2-octyl-3-hydroxy-4-fluorophenyl)octanoic acid amide,
5-[2-(2-allyl-3-chloro-4-acetylphenylthio)ethylsulfonyl]tetrazole,
5-(2-propyl-3,4-dichlorophenoxy)pentanoic acid,
5-(2-methyl-3,4-difluorobenzyloxy)-1-pentanol,
S-[10-(2-hexyl-3-chloro-4-fluorophenylsulfinyl)decyl]isothiuronium bromide,
5-[6-(2-propyl-3-chloro-4-butanoylphenyl)hex-5-enylthio]-tetrazole,
N,N-diethyl-6-(2-ethyl-3-hydroxy-4-iodophenoxy)undecanoic acid amide,
4-(2-propyl-3,4-dichlorobenzyloxy)butanoic acid,
N-(7-[2-isopropyl-3-iodo-4-(4-methoxybenzoyl)benzylthio]octyl)morpholine,
5-[2-(2-propyl-3,4-dichlorobenzyloxy)ethylthio]-tetrazole,
5-(2-allyl-3-fluoro-4-iodophenyl)-3-methyl-3-benzylpentanoic acid,
5-[4-(2-methyl-3-bromo-4-propionyl-α-methylbenzyloxy)butyl]-tetrazole,
5-[3-(2-propyl-3,4-dichlorobenzyloxy)propyl]tetrazole,
5-[6-(2-methyl-3,4-diiodobenzylsulfinyl)hexyloxy]-tetrazole,
6-(2-propyl-3,4-dichlorophenyl)hexanoic acid,
5-[3-(2-ethyl-3,4-dichlorophenyl)prop-2-enyl]tetrazole,
5-(3-(2-nonyl-3-hydroxy-4-fluorophenyl)hexylsulfonyl]tetrazole,
5-[4-(2-isopropyl-3,4-dichlorophenoxy)butylamino]-tetrazole,
5-[3-(2-butyl-3-iodo-4-fluorobenzylthio)propylthio]-tetrazole,
5-[3-(2-propyl-3,4-dichlorobenzylamino)propylthio]-tetrazole,
5-[4-(2-[3-hydroxypropyl]-3,4-dichlorobenzyloxy)butyl]-tetrazole,
5-[N-methyl-3-(2-methyl-3,4-difluorophenyl)propylamino]-tetrazole,
5-[4-(2-propyl-3,4-dichlorophenoxy)butyl]tetrazole,
5-[3-(2-butenyl-3-hydroxy-4-chloro-α-ethylbenzyloxy)propylsulfonyl]-tetrazole,
5-[5-(2-propyl-3,4-dichlorophenyl)pentyl]tetrazole,
10-(2-propyl-3,4-dichlorophenyl)undecanoic acid,
4-(2-butyl-3-iodo-4-bromophenyl)-3-phenylhexanoic acid,
8-(2-allyl-3-hydroxy-4-chlorophenyl)-7,8-dimethyldecanoic acid amide,
3-(2-propyl-3,4-dichloro-α-methylbenzylthio)propionic acid,
4-(2-allyl-3-chloro-4-cyclohexanoylphenyl)pentanoic acid,
methyl 3-[2-(3-hydroxypropyl)-3-bromo-4-fluorophenylsulfonyl]octanoate
5-(4-[2-propyl-3-chloro-4-(4-chlorobenzoyl)benzyloxy]butylamino)-tetrazole, and
4-(2-propyl-3,4-dichlorophenyl)-3-butenoic acid.

The following examples further illustrate the preparation of the starting materials, intermediates, and compounds of this invention. The examples are illustrative only and are not intended to limit the scope of the invention. Where structures were confirmed by proton nuclear magnetic resonance analysis, the compound is so designated by "NMR".

## Example 1

## 6-(2-Propyl-3,4-dichlorophenoxy)hexanoic acid

A. Preparation of 2-allyl-3,4-dichlorophenol

A solution of 100 g of 3,4-dichlorophenol, 50 ml of allyl bromide, 84 g of potassium carbonate, and 1.0 g of potassium iodide in 500 ml of methyl ethyl ketone was allowed to reflux overnight. The solution was then cooled, filtered, and evaporated to dryness. The residue was then heated at 200°C for approximately four hours. NMR analysis indicated that the 113.6 g of remaining material was primarily the desired 2-allyl-3,4-dichlorophenol.

B. Preparation of 2-propyl-3,4-dichlorophenol

A solution of 117.5 g of 2-allyl-3,4-dichlorophenol in 880 ml of toluene was treated with about 8 g of Raney nickel and hydrogenated at room temperature for three hours. The solution was filtered and evaporated. Purification of the residue using high pressure liquid chromatography (silica gel eluting with 4% ethyl acetate in hexane) provided the desired title compound together with small amounts of 2-propyl-4,5-dichlorophenol. NMR.

C. Preparation of 6-(2-propyl-3,4-dichlorophenoxy)hexanoic acid

To a solution of 1.1 g of 2-propyl-3,4-dichlorophenol in 30 ml of tetrahydrofuran and 30 ml of hexamethylphosphoramide were added 4.1 g of 6-bromohexanoic acid. To the solution were then added 1.3 g of a 50% oil dispersion of sodium hydride. A catalytic amount of potassium iodide was added and the

reaction was stirred under a nitrogen atmosphere at 60—70°C overnight. The reaction was cooled to room temperature and ethyl acetate and water were added. The solution was evaporated to dryness. Fresh ethyl acetate and water were added and the layers were separated. Fresh ethyl acetate was added to the aqueous layer and the aqueous layer was adjusted to pH 2. The layers were separated and the organic layer was washed with a saturated sodium chloride solution. The organic layer was then filtered through sodium sulfate and evaporated to dryness. The resulting residue was extracted with hexane and evaporated to dryness to afford 500 mg of the title compound.

Example 2

5-[4-(2-Propyl-3,4-dichlorophenoxy)butyl]tetrazole

A. Preparation of 4-(2-propyl-3,4-dichlorophenoxy)pentane nitrile

To a solution of 37.1 g of 2-propyl-3,4-dichlorophenol in 400 ml of dry tetrahydrofuran and 25 ml of hexamethylphosphoramide were added 32 ml of 5-bromovaleronitrile. Sodium hydride (7.2 g of a 60% dispersion in mineral oil) was added and the reaction was stirred for about three days at 65°C. The reaction mixture was cooled to room temperature and ethyl acetate and water were added. The solution was evaporated and ethyl acetate and water were added. The layers were separated and the ethyl acetate was washed several times with dilute hydrochloric acid. The organic layer was filtered through sodium sulfate and evaporated to provide the desired nitrile intermediate. NMR.

B. Preparation of 5-[4-(2-propyl-3,4-dichlorophenoxy)butyl]-tetrazole

To a solution of 10.2 g of 4-(2-propyl-3,4-dichlorophenoxy)pentane nitrile in 80 ml of dimethylformamide were added 23.27 g of sodium azide and 18.97 g of ammonium chloride. The reaction was stirred for about two days at 120°C under a nitrogen atmosphere. The reaction mixture was cooled to room temperature and ethyl acetate and water were added. The pH was adjusted to 2 and the layers were separated. The ethyl acetate layer was washed several times with a saturated sodium chloride solution, filtered through sodium sulfate, and evaporated to dryness. Trituration of the residue with hexane followed by filtration provided 47.9 g of the title product. NMR.

Analysis: $C_{14}H_{18}Cl_2N_4O$;

| | | | | | |
|---|---|---|---|---|---|
| Calc.: | C, 51.07; | H, 5.51; | N, 17.02; | O, 4.86; | Cl, 21.54; |
| Found: | C, 50.58; | H, 5.54; | N, 18.42; | O, 5.56; | Cl, 20.24. |

Example 3

5-[5-(2-Propyl-3,4-dichlorophenoxy)pentyl]tetrazole

A. Preparation of 5-(2-propyl-3,4-dichlorophenoxy)pentyl bromide

To a solution of 3.12 g of 2-propyl-3,4-dichlorophenol in 30 ml of dry tetrahydrofuran and 30 ml of dry hexamethylphosphoramide were added 0.72 g of a 50% sodium hydride dispersion in oil. Two milliliters of 1,5-dibromopentane were then added followed by a catalytic amount of potassium iodide. The reaction was stirred overnight under a nitrogen atmosphere at 60—70°C. The reaction was then cooled to room temperature, ethyl acetate and water were added, and the solution was evaporated to dryness. Ethyl acetate and water were added to the residue and the layers were separated. The ethyl acetate layer was washed with dilute acid to remove residual hexamethylphosphoramide. The ethyl acetate was then washed with a saturated sodium chloride solution, filtered through sodium sulfate, and evaporated to dryness. The residue was purified by high pressure liquid chromatography to provide 1.6 g of the desired bromo intermediate. NMR.

B. Preparation of 5-(2-propyl-3,4-dichlorophenoxy)hexane nitrile

A solution of 1.6 g of 5-(2-propyl-3,4-dichlorophenoxy)pentyl bromide and 0.22 g of sodium cyanide were heated overnight at 75—85°C in 50 ml of dimethylformamide. The solution was evaporated *in vacuo* and ethyl acetate and water were added to the residue. The layers were separated and the organic layer was washed several times with a saturated sodium chloride solution. The organic solution was filtered through sodium sulfate and evaporated to dryness to give 1.38 g of the desired sub-title intermediate. NMR.

C. Preparation of 5-[5-(2-propyl-3,4dichlorophenoxy)pentyl]-tetrazole

Following the procedure of Example 2B, 1.34 g of 5-(2-propyl-3,4-dichlorophenoxy)hexane nitrile were transformed into the desired title product. NMR.

Analysis: $C_{15}H_{20}Cl_2N_4O$;

| | | | | | |
|---|---|---|---|---|---|
| Calc.: | C, 52.59; | H, 5.87; | N, 16.32; | O, 4.66; | Cl, 20.66; |
| Found: | C, 52.83; | H, 5.08; | N, 16.03; | O, 4.86; | Cl, 20.43. |

Example 4

5-[6-(2-Propyl-3,4-dichlorophenoxy)hexyl]tetrazole

Following the procedure of Example 3, 7.8 g of 2-propyl-3,4-dichlorophenol and 5.9 ml of 1,6-dibromohexane were transformed into 3.16 g of the title product. NMR.

Analysis: $C_{16}H_{22}Cl_2N_4O$;

Calc.: C, 53.72; H, 6.21; N, 15.68; O, 4.48; Cl, 19.85;
Found: C, 53.89; H, 6.18; N, 15.72; O, 4.69; Cl, 20.11.

Example 5

5-[4-(2-Propyl-3-chloro-4-acetylphenoxy)butyl]tetrazole

Following the procedure of Example 1A, 50 g of 3-chlorophenol and 32 ml of allyl bromide were heated with 42 g of potassium carbonate and a catalytic amount of potassium iodide in 250 ml of methyl ethyl ketone. After evaporation the residue was heated to approximately 200°C for 2—3 hours to give 62.8 g of a mixture of 2- and 6-allyl-3-chlorophenol. Following Example 1B, this mixture was hydrogenated in the presence of 7 g of Raney nickel in 530 ml of toluene. Chromatography of the resulting products provided both the desired 2-propyl-3-chlorophenol and the by-product 2-propyl-5-chlorophenol.

Five and seven-tenths grams of 2-propyl-3-chlorophenol were then reacted with 5.9 ml of 5-bromovaleronitrile following the procedure of Example 2A. The resulting 4-(2-propyl-3-chlorophenoxy)pentane nitrile was provided by chromatography. This purified nitrile intermediate (3.3 g) was dissolved in about 65 ml of methylene chloride to which 3.0 g of aluminum chloride and 1.7 ml of acetyl chloride had been added and the reaction was heated to reflux overnight. The reaction was cooled, additional methylene chloride was added, and the mixture was poured into ice/hydrochloric acid. The layer were separated and the organic layer was washed first with an aqueous sodium bicarbonate solution and then with water. The organic solution was dried over sodium sulfate and evaporated to dryness. Purification by chromatography provided 2.67 g of the desired 4-(2-propyl-3-chloro-4-acetylphenoxy)pentane nitrile. NMR.

A solution of 0.5 g of 4-(2-propyl-3-chloro-4-acetylphenoxy)pentane nitrile and 1.3 g of tetramethylguanidinium azide in 3 ml of dimethylformamide was heated overnight at 125°C. The reaction was cooled, ethyl acetate and water were added, and the layers were separated. Water was added to the ethyl acetate layer, the water layer was acidified, and the layers were separated. The ethyl acetate was dried and evaporated to dryness. The residue was purified by chromatography to give the desired title product. NMR.

Analysis: $C_{16}H_{21}ClN_4O_2$;
Calc.: C, 57.06; H, 6.28; N, 16.63;
Found: C, 58.58; H, 6.57; N, 14.71.

Example 6

5-[6-(2-Octyl-3,4-dichlorophenoxy)hexyl]tetrazole

A. Preparation of 2,3-dihydro-2-hydroxy-4,5-dichlorobenzofuran

A solution of 20.3 g of 2-allyl-3,4-dichlorophenol in methylene chloride was ozonized for 30 minutes at −78°C. The solution was warmed to −23°C and ozonized an additional ten minutes. Dimethyl sulfide was added to the reaction and the solution was then evaporated to dryness. Purification by high pressure liquid chromatography afforded 1.3 g of 2,3-dihydro-2-hydroxy-4,5-dichlorobenzofuran as white crystals.

B. Preparation of 2-(2-octenyl)-3,4-dichlorophenol

Twenty-five grams of n-hexyltriphenylphosphonium bromide were dissolved in 350 ml of dry tetrahydrofuran and cooled to −10°C. To the solution were added 37 ml of a 1.6 M solution of n-butyllithium in hexane. The solution was slowly warmed to room temperature and stirred for one hour. The solution was then cooled to 0°C and a solution of 2.38 g of 2,3-dihydro-2-hydroxy-4,5-dichlorobenzofuran in 20 ml of tetrahydrofuran was added. The reaction was allowed to reflux for approximately 18 hours. The solution was then cooled and evaporated to dryness. Ethyl acetate and water were added and the pH was adjusted to 2.5. The layers were separated. The organic layer was washed with a saturated sodium chloride solution, filtered through sodium sulfate, and evaporated to dryness. The residue was purified by high pressure liquid chromatography giving 2.51 g of the desired intermediate as a mixture of cis and trans isomers.

C. Preparation of 2-octyl-3,4-dichlorophenol

A solution of 1.08 g of 2-(2-octenyl)-3,4-dichlorophenol in 50 ml of toluene was hydrogenated in the presence of Raney nickel. The catalyst was removed by filtration and the solvent was evaporated to dryness affording 1.04 g of 2-octyl-3,4-dichlorophenol. NMR.

D. Preparation of 5-[6-(2-octyl-3,4-dichlorophenoxy)hexyl]-tetrazole

Following the procedures of Example 3, 775 mg of 2-octyl-3,4-dichlorophenol and 0.43 ml of 1,6-dibromohexane were transformed into 10.6 mg of the title product. The NMR spectrum was consistent with the structure of the desired product.

Example 7

5-[5-(2-Dodecyl-3,4-dichlorophenoxy)pentyl]tetrazole

Following the general procedures of Example 6, 2,3-dihydro-2-hydroxy-4,5-dichlorobenzofuran and n-decyltriphenylphosphonium bromide were transformed into the title product. The NMR spectrum was consistent with the structure of the desired product.

Example 8

5-[3-(2-Propyl-3,4-dichlorophenoxy)propanethio]tetrazole

Following the procedure of Example 3A, 3-(2-propyl-3,4-dichlorophenoxy)propyl bromide was prepared from 2-propyl-3,4-dichlorophenol and 1,3-dibromopropane. A solution of 1.96 g of 3-(2-propyl-3,4-dichlorophenoxy)propyl bromide, 1.66 g of potassium carbonate, and 0.615 g of 5-mercaptotetrazole in 20 ml of dimethylformamide was stirred overnight at room temperature. Ethyl acetate and water were added and the layers were separated. The organic layer was washed successively with dilute acid, dilute potassium carbonate solution, and again with dilute acid. The organic solution was dried over sodium sulfate and evaporated to dryness. The residue was washed several times with water to provide 295 mg of the desired title product. NMR.

Analysis: $C_{13}H_{16}Cl_2N_4OS$;

| | | | | |
|---|---|---|---|---|
| Calc.: | C, 44.96; | H, 4.64; | N, 16.13; | S, 9.23; |
| Found: | C, 45.12; | H, 4.57; | N, 16.14; | S, 9.41. |

Example 9

6-(2-Pentyl-3,4-dichlorophenoxy)hexanoic acid

Following the general procedure of Example 6B, 2.3 g of 2,3-dihydro-2-hydroxy-4,5-dichlorobenzofuran and 17.3 g of propyltriphenylphosphonium bromide were reacted to provide 840 mg of 2-(2-pentenyl)-3,4-dichlorophenol which upon hydrogenation following the procedure of Example 6C provided 300 mg of 2-pentyl-3,4-dichlorophenol. This phenol was then reacted with 5.1 mmoles of 6-bromohexanoic acid following the procedure of Example 1C to provide 58 mg of the desired title product. NMR.

Example 10

6-[2-(2-Hydroxyethyl)-3,4-dichlorophenoxy]hexanoic acid

A. Preparation of 2-(2-hydroxyethyl)-3,4-dichlorophenol

A solution of 2.26 g of 2,3-dihydro-2-hydroxy-4,5-dichlorobenzofuran in 15 ml of absolute ethanol was cooled to 0°C by means of an external ice bath. Three millimoles (114 mg) of sodium borohydride were added to the solution and the reaction was stirred while allowing the reaction to warm to room temperature. The mixture was evaporated under reduced pressure and water and ethyl acetate were added to the residue. The pH of the aqueous layer was adjusted to 2.5 with the addition of acid and the layers were separated. The ethyl acetate layer was dried and evaporated to provide the subtitle intermediate phenol which was used without further purification.

B. Preparation of 6-[2-(2-hydroxyethyl)-3,4-dichlorophenoxy]hexanoic acid

Following the procedure of Example 1C, two millimoles each of 2-(2-hydroxyethyl)-3,4-dichlorophenol and 6-bromohexanoic acid were reacted to provide 55 mg of the desired title product which crystallized from methylene chloride.

Example 11

5-[4-(2-Propyl-3-hydroxy-4-chlorophenoxy)butyl]-tetrazole

A. Preparation of 5-[4-(2-propyl-3-hydroxyphenoxy)butyl]-tetrazole

Six grams of 2-propylresorcinol and 6.4 g of 5-bromovaleronitrile were reacted following the procedure of Example 2A to provide 3.4 g of the desired nitrile intermediate. This intermediate was heated to 125°C overnight with 40 mmoles of tetramethylguanidinium azide in 10 ml of dimethylformamide. The solvent was removed under reduced pressure and the residue was dissolved in ethyl acetate. The organic solution was washed with water adjusted to pH 6. The organic layer was dried and evaporated to yield the desired subtitle tetrazole intermediate which crystallized after evaporation of the solvent. NMR.

B. Preparation of 5-[4-(2-propyl-3-hydroxy-4-chlorophenoxy)butyl]-tetrazole

A solution of 1.38 g of 5-[4-(2-propyl-3-hydroxyphenoxy)butyl]-tetrazole in 25 ml of tetrahydrofuran was cooled to −10°C by means of an external alcohol-ice bath. One millimole of sulfuryl chloride was slowly added and the reaction mixture was stirred for one hour. The mixture was evaporated under reduced pressure and the residue was triturated with diethyl ether. The ether was evaporated and the residue was purified by reverse phase high pressure liquid chromatography. The appropriate fractions provided 42 mg of the desired title product plus 92 mg of the 2-propyl-3-hydroxy-6-chlorophenoxy isomer and a small amount of the 2-propyl-3-hydroxy-4,6-dichlorophenoxy compound.

Example 12

7-(2-Propyl-3,4-dichlorophenoxy)heptanoic acid

A solution of 3.09 g of 2-propyl-3,4-dichlorophenol, 3.1 g of 7-bromoheptanoic acid, 3.1 g of potassium carbonate, and 2.0 g of potassium iodide in 50 ml of methyl ethyl ketone was heated at reflux overnight. The reaction mixture was cooled to room temperature and evaporated *in vacuo*. Ethyl acetate and water were added to the residue and the layers were separated. Fresh water was added to the organic layer, the pH was adjusted to 2.0 with acid, and the layers were separated. The organic layer was washed with a saturated sodium chloride solution, filtered through sodium sulfate, and evaporated to dryness to give

4.68 g of the desired title product.

Analysis: $C_{16}H_{22}Cl_2O_3$;

Calc.: C, 57.67; H, 6.65; O, 14.40; Cl, 21.28;

Found: C, 55.85; H, 6.59; O, 15.81; Cl, 20.33.

The compounds of Formula I should be useful in treating any condition, including clinical conditions, which is characterized by excessive release of leukotrienes $C_4$, $D_4$, or $E_4$. These conditions include immediate type hypersensitivity reactions such as asthma. Evidence obtained over the past few years has shown the presence of leukotrienes in sputum of patients with chronic bronchitis (Turnbull, *et al., Lancet II*, 526 (1977)) and cystic fibrosis (Cromwell, *et al., Lancet II*, 164 (1981)), suggesting a role of leukotrienes in the pathology of those diseases. Furthermore, Lewis and colleagues [*Int. J. Immunopharmacology 4*, 85 (1982)] have recently detected material in rheumatoid synovial fluid that reacts antigenically with antibody to $LTD_4$. This may hallmark the existence of leukotriene permeability factors that, together with $LTB_4$, augment the inflammatory process in the diseased joints. Therefore, the compounds described in this invention should also alleviate some of the symptoms of chronic bronchitis and cystic fibrosis and possibly rheumatoid arthritis by virtue of their ability to antagonize leukotrienes.

The term "excessive release" of leukotrienes refers to an amount of leukotrienes sufficient to cause the particular condition associated with such amount. The amount of leukotriene which is considered to be excessive will depend on a variety of factors, including the specific leukotriene(s) involved, the amount of leukotriene required to cause the particular condition, and the species of the mammal involved. As will be appreciated by those skilled in the art, the success of treating a mammal suffering from or susceptible to a condition characterized by an excessive release of leukotrienes with a compound of Formula I will be measured by the regression or prevention of the symptoms of the condition.

Leukotriene antagonism was demonstrated by the following test procedure:

Male, Hartley guinea pigs weighing 200—450 grams were killed by decapitation. A section of terminal ileum was removed, the lumen cleaned, and the tissue divided into 2.5 cm segments. The ilea were mounted in 10 ml tissue baths containing Krebsbicarbonate solution of the following composition in mmoles/liter KCl, 4.6; $CaCl_2 \cdot 2H_2O$, 1.2; $KH_2PO4$, 1.2; $MgSO_4 \cdot 7H_2O$, 1.2; NaCl, 118.2; $NaHCO_3$, 24.8; and dextrose, 10.0. The bath fluid was maintained at 37°C and aerated with 95 percent oxygen and 5 percent $CO_2$. In addition, the buffer contained 1 × 10—6 M atropine to reduce ileal spontaneous activity. Isometric measurements were made with a Grass FT03C force-displacement transducer and recorded on a Grass polygraph as change in grams of force. A passive force of 0.5 g was applied to the tissues. After an appropriate equilibration period, single submaximal control responses to pure $LTD_4$ were obtained. Following a five minute exposure of the ileum to an experimental drug, the control concentration of $LTD_4$ was added to the tissue bath. The response of the ileum to $LTD_4$ in the presence of the drug was compared to the response in the absence of the drug.

For some of the drugs in this series a more detailed analysis of $LTD_4$ antagonism was made. In these experiments, cumulative concentration-response curves were obtained to $LTD_4$ in guinea pig ileum and trachea. This was followed by a 30 minute incubation with various concentrations of the experimental drug. The concentration response curve to $LTD_4$ was then repeated in the presence of the antagonist. Only one concentration of antagonist was used on a single tissue. KB values were calculated by the method of Furchgott [*Ann. N.Y. Acad. Sci., 139*, 553 (1967)] using the following equation.

$$K_B = \frac{[\text{Antagonist}]}{\text{Dose Ratio} - 1}$$

Dose ratio refers to the concentration of agonist required to elicit 50 percent of the maximal response ($ED_{50}$) in the presence of the antagonist divided by the $ED_{50}$ in the absence of the antagonist. Calculations were performed with the aid of a computer and a digital plotter. The $pA_2$ is then calculated as the negative log of $K_B$ when the slope of the Schild plot is not significantly different from unity.

The testing of the compounds of Formula I in these two test procedures is summarized in Table I.

TABLE I
Percent inhibition of $LTD_4$ evoked ileal contractions

| Compound of Example No. | Compound concentration | | | | |
|---|---|---|---|---|---|
| | $3 \times 10^{-6}$M | $1 \times 10^{-6}$M | $3 \times 10^{-7}$M | $1 \times 10^{-7}$M | pA2 |
| 1 | 78 | 51 | | | |
| 2 | | 94 | 70 | 56 | 7.0 |
| 3 | 92 | 75 | 51 | | 6.6* |
| 4 | 96 | 84 | | | 7.0* |
| 5 | | | 73 | 49 | 6.98* |
| 6 | 59 | 53 | | | |
| 7 | 16 | | | | |
| 8 | | | 79 | 59 | |
| 9 | 57 | 21 | | | |
| 10 | 22 | | | | |
| 11 | 20 | | | | 5.5* |
| 12 | 31 | | | | |

* estimated.

The compounds or formulations of the present invention may be administered by the oral and rectal routes, topically, parenterally, *e.g.,* by injection and by continuous or discontinuous intra-arterial infusion, in the form of, for example, tablets, lozenges, sublingual tablets, sachets, cachets, elixirs, suspensions, aerosols, ointments, for example, containing from 1 to 10% by weight of the active compound in a suitable base, soft and hard gelatin capsules, suppositories, injectable solutions and suspensions in physiologically acceptable media, and sterile packaged powders adsorbed onto a support material for making injectable solutions. Advantageously for this purpose, compositions may be provided in dosage unit form, preferably each dosage unit containing from about 5 to 500 mg. (from about 5 to 50 mg. in the case of parenteral or inhalation administration, and from about 25 to 500 mg. in the case of oral or rectal administration) of a compound of Formula I. Dosages of from about 0.5 to 300 mg./kg. per day, preferably 0.5 to 20 mg./kg., of active ingredient may be administered although it will, of course, readily be understood that the amount of the compound or compounds of Formula I actually to be administered will be determined by a physician, in the light of all the relevant circumstances including the condition to be treated, the choice of compound to be administered and the choice of route of administration and therefore the above preferred dosage range is not intended to limit the scope of the present invention in any way.

The formulations of the present invention normally will consist of at least one compound of Formula I mixed with a carrier, or diluted by a carrier, or enclosed or encapsulated by an ingestible carrier in the form of a capsule, sachet, cachet, paper or other container or by a disposable container such as an ampoule. A carrier or diluent may be a solid, semisolid or liquid material which serves as a vehicle, excipient or medium for the active therapeutic substance.

Some examples of the diluents or carrier which may be employed in the pharmaceutical compositions of the present invention are lactose, dextrose, sucrose, sorbitol, mannitol, propylene glycol, liquid paraffin, white soft paraffin, kaolin, fumed silicon dioxide, microcrystalline cellulose, calcium silicate, silica, polyvinylpyrrolidone, cetostearyl alcohol, starch, modified starches, gum acacia, calcium phosphate, cocoa butter, ethoxylated esters, oil of theobroma, arachis oil, alginates, tragacanth, gelatin, syrup, methyl cellulose, polyoxyethylene sorbitan monolaurate, ethyl lactate, methyl and propyl hydroxybenzoate, sorbitan trioleate, sorbitan sesquioleate and oleyl alcohol and propellants such as trichloromonofluoromethane, dichlorodifluoromethane and dichlorotetrafluoroethane. In the case of tablets, a lubricant may be incorporated to prevent sticking and binding of the powdered ingredients in the dies and on the punch of the tableting machine. For such purposes there may be employed for instance aluminum, magnesium or

calcium stearate, talc or mineral oil.

Preferred pharmaceutical forms of the present invention are capsules, tablets, suppositories, injectable solutions, creams and ointments. Especially preferred are formulations for inhalation application, such as an aerosol, and for oral ingestion.

**Claims for the Contracting States: BE CH DE FR GB IT LI LU NL SE**

1. A compound of the formula

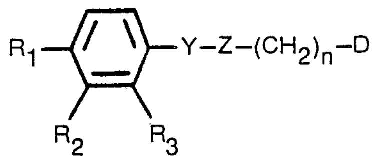

I

or a pharmaceutically acceptable salt thereof, wherein:

$R_1$ is $R_5-\overset{O}{\overset{\|}{C}}-$ or halo;

$R_2$ is halo or hydroxy;

$R_3$ is $C_1$—$C_{12}$ alkyl, hydroxy-substituted $C_1$—$C_{12}$ alkyl, or $C_2$—$C_6$ alkenyl;

Y is —O—, $-\overset{(O)_p}{\overset{\|}{S}}-$, or $-CR_6R_7-$,

Z is —O—, $-\overset{(O)_p}{\overset{\|}{S}}-$, or $-CR_8R_9-$,

or when taken together, —Y—Z is —CH=CH—;

n is 1—10;

D is CN, SCN or $QR_4$;

Q is —O—, —NR—, $-\overset{(O)_p}{\overset{\|}{S}}-$ or a bond;

$R_4$ is $-COR_{10}$, hydroxy, $-NR_{11}R_{12}$, $-SC(=NH)NH_2$, or

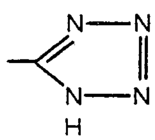

;

where

R is hydrogen or $C_1$—$C_3$ alkyl;

$R_5$ is hydrogen, $C_1$—$C_6$ alkyl, $C_3$—$C_8$ cycloalkyl, or phenyl optionally substituted with halo, $C_1$—$C_4$ alkyl, or $C_1$—$C_4$ alkoxy;

each of $R_6$ and $R_7$ is independently hydrogen, $C_1$—$C_{10}$ alkyl, phenyl, or benzyl;

$R_8$ and $R_9$ are hydrogen;

$R_{10}$ is hydroxy, $C_1$—$C_4$ alkoxy, —NHOH, or $-NR_{11}R_{12}$;

each of $R_{11}$ and $R_{12}$ is independently hydrogen, $C_1$—$C_3$ alkyl, or when taken together with the nitrogen atom form a morpholine or N-methyl piperazine ring; and

p is 0, 1, or 2;

with the provisions that:

a) when $R_1$ is 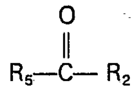 may not be hydroxy;

b) when one of Y and Z is —O— or

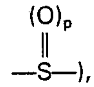

the other of Y and Z may not be —O— or

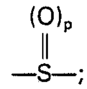

and

c) when $R_4$ is $COR_{10}$, hydroxy, $—NR_{11}R_{12}$, or $—SC(=NH)NH_2$, Q may only be a bond.

2. A compound of formula (I) wherein D is $QR_4$.

3. A compound of claim 1 which is
5-(2-propyl-3,4-dichlorophenoxy)pentanoic acid or a pharmaceutically acceptable salt thereof, or
4-(2-propyl-3,4-dichlorobenzyloxy)butanoic acid or a pharmaceutically acceptable salt thereof, or
6-(2-propyl-3,4-dichlorophenyl)hexanoic acid or a pharmaceutically acceptable salt thereof, or
5-[4-(2-propyl-3,4-dichlorophenoxy)butyl]tetrazole or a pharmaceutically acceptable salt thereof, or
5-[3-(2-propyl-3,4-dichlorobenzyloxy)propyl]tetrazole or a pharmaceutically acceptable salt thereof, or
5-[5-(2-propyl-3,4-dichlorophenyl)pentyl]tetrazole or a pharmaceutically acceptable salt thereof, or
5-[3-(2-propyl-3,4-dichlorophenoxy)propylthio]-tetrazole or a pharmaceutically acceptable salt thereof, or
5-[2-(2-propyl-3,4-dichlorobenzyloxy)ethylthio]-tetrazole or a pharmaceutically acceptable salt thereof, or
5-[4-(2-propyl-3,4-dichlorophenyl)butylthio]-tetrazole or a pharmaceutically acceptable salt thereof.

4. A pharmaceutical formulation comprising a compound of formula (I) as defined in any one of claims 1—3, provided D is $QR_4$, in association with one or more pharmaceutically acceptable carriers or diluents therefore.

5. A process for preparing a compound of formula (I) which comprises

(a) reacting a compound of formula (II)

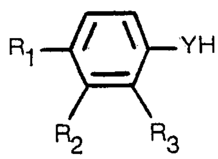

(II)

wherein $R_1$, $R_2$, and $R_3$ are as defined in formula (I), and Y is S or O, with a compound of formula (III)

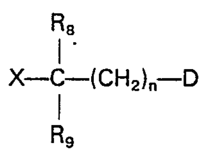

(III)

wherein $R_8$, $R_9$, and n are as defined in formula (I), X is a leaving group, and D is CN, SCN, or $QR_4$ as defined in formula (I) to provide a compound of formula (I) wherein Y is S or O; or

(b) reacting a compound of the formula

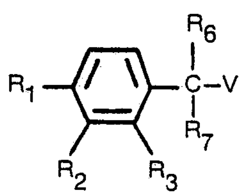

wherein $R_1$, $R_2$, $R_3$, $R_6$, and $R_7$ are as defined in formula (I) with a compound of the formula

$$W—(CH_2)_n—D$$

wherein n is as defined in formula (I), D is CN, SCN, or $QR_4$ as defined in formula (I), and one of V and W is a

leaving group and the other is —ZH, where Z is O or S to provide a compound of formula (I) wherein Z is O or S; or

(c) reacting a compound of formula (XIII)

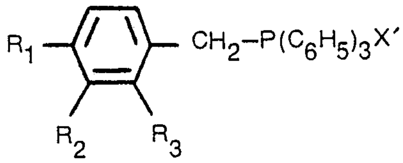

XIII

wherein $R_1$, $R_2$, and $R_3$ are as defined in formula (I) and X′ is halo, with a compound of formula (XIV)

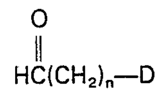

wherein n is as defined in formula (I) and D is CN, SCN, or $QR_4$ as defined in formula (I), to provide a compound of formula (I) wherein Y—Z is —CH=CH—; or

(d) reacting a compound of formula (XVI)

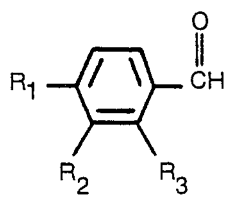

XVI

wherein $R_1$, $R_2$, and $R_3$ are as defined in formula (I), with a compound of the formula (XVII)

$$X'(C_6H_5)_3P—(CH_2)_{n+1}—D \qquad (XVII)$$

wherein n is as defined in formula (I), X′ is halo, and D is CN, SCN, or $QR_4$ as defined in formula (I) to provide a compound of formula (I) wherein Y—Z is —CH=CH—; or

(e) reacting a compound of formula (I) wherein D is CN with an alkali metal azide and ammonium chloride, or with tetramethylquanidinium azide to provide a compound of formula (I) wherein D is 5-tetrazolyl; or

(f) reacting a compound of formula IV

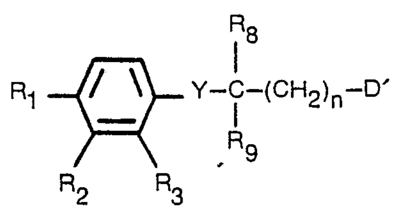

IV

wherein $R_1$, $R_2$, $R_3$, $R_9$, $R_8$, and n are as defined in formula (I) Y is O or S, and D′ is halo, with an alkali metal cyanide or an alkali metal thiocyanate to provide a compound of formula (I) wherein D is CN or SCN; or

(g) reacting a compound of formula IV wherein D′ is halo with 5-mercaptotetrazole to provide a compound of formula (I) wherein D is thiotetrazole; or

(h) oxidizing a compound of formula (I) wherein Y or Z is S or SO to provide the corresponding compound of formula (I) wherein Y or Z is SO or $SO_2$; and

(i) optionally salifying the resulting compound of formula (I).

6. A compound of formula (I) as claimed in any one of claims 1—3, provided D is $QR_4$ for use in treating or preventing any condition characterized by excessive release of leukotrienes, or for treating or preventing immediate hypersensitivity reaction.

7. A compound of formula (I) in which $R_1$, $R_2$, $R_3$, Y, Z, n and D are as defined in claim 1, provided D is $QR_4$ and provided each of $R_8$ and $R_9$ is independently hydrogen, $C_1$—$C_{10}$ alkyl, phenyl, or benzyl, for the manufacture of a medicament for treating or preventing any condition characterised by excessive release of leukotrienes, or for treating or preventing immediate hypersensitivity reaction.

**Claims for the Contracting State: AT**

1. A process for preparing a compound of the formula

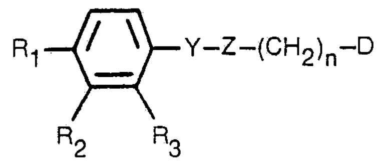

or a pharmaceutically acceptable salt thereof, wherein:

$R_1$ is

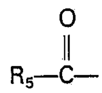 or halo;

$R_2$ is halo or hydroxy;

$R_3$ is $C_1$—$C_{12}$ alkyl, hydroxy-substituted $C_1$—$C_{12}$ alkyl, or $C_2$—$C_6$ alkenyl;

Y is —O—,

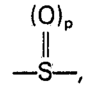, or —$CR_6R_7$—,

Z is —O—,

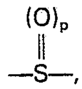, or —$CR_8R_9$—,

or when taken together, —Y—Z is —CH=CH—;

n is 1—10;

D is CN, SCN or $QR_4$;

Q is —O—, —NR—,

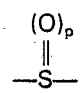 or a bond;

$R_4$ is —$COR_{10}$, hydroxy, —$NR_{11}R_{12}$, —SC(=NH)$NH_2$, or

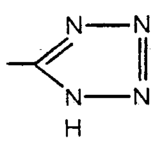 ;

where

R is hydrogen or $C_1$—$C_3$ alkyl;

$R_5$ is hydrogen, $C_1$—$C_6$ alkyl, $C_3$—$C_8$ cycloalkyl, or phenyl optionally substituted with halo, $C_1$—$C_4$ alkyl, or $C_1$—$C_4$ alkoxy;

each of $R_6$ and $R_7$ is independently hydrogen, $C_1$—$C_{10}$ alkyl, phenyl, or benzyl;

$R_8$ and $R_9$ are hydrogen;

$R_{10}$ is hydroxy, $C_1$—$C_4$ alkoxy, —NHOH, or —$NR_{11}R_{12}$;

each of $R_{11}$ and $R_{12}$ is independently hydrogen, $C_1$—$C_3$ alkyl, or when taken together with the nitrogen atom form a morpholine or N-methyl piperazine ring; and

p is 0, 1, or 2;

with the provisions that:

a) when $R_1$ is

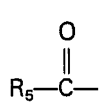 $R_2$ may not be hydroxy;

b) when one of Y and Z is —O— or

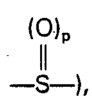),

the other of Y and Z may not be —O— or

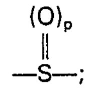

and

c) when $R_4$ is $COR_{10}$, hydroxy, $—NR_{11}R_{12}$, or $—SC(=NH)NH_2$, Q may only be a bond, which comprisės

(a) reacting a compound of formula (II)

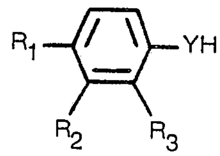

(II)

wherein $R_1$, $R_2$, and $R_3$ are as defined in formula (I), and Y is S or O, with a compound of formula (III)

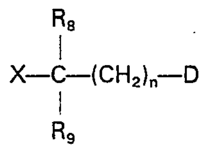

(III)

wherein $R_8$, $R_9$, and n are as defined in formula (I), X is a leaving group, and D is CN, SCN, or $QR_4$ as defined in formula (I) to provide a compound of formula (I) wherein Y is S or O; or

(b) reacting a compound of the formula

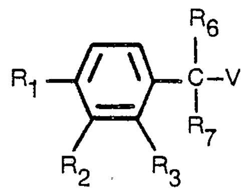

wherein $R_1$, $R_2$, $R_3$, $R_6$, and $R_7$ are as defined in formula (I) with a compound of the formula

$$W—(CH_2)_n—D$$

wherein n is as defined in formula (I), D is CN, SCN, or $QR_4$ as defined in formula (I), and one of V and W is a leaving group and the other is —ZH, where Z is O or S to provide a compound of formula (I) wherein Z is O or S; or

(c) reacting a compound of formula (XIII)

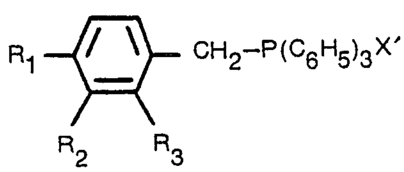

XIII

wherein $R_1$, $R_2$, and $R_3$ are as defined in formula (I) and X′ is halo, with a compound of formula (XIV)

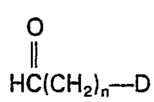

wherein n is as defined in formula (I) and D is CN, SCN, or $QR_4$ as defined in formula (I), to provide a compound of formula (I) wherein Y—Z is —CH=CH—; or

(d) reacting a compound of formula (XVI)

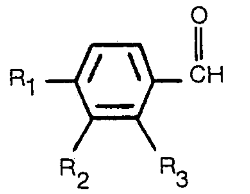

XVI

wherein $R_1$, $R_2$, and $R_3$ are as defined in formula (I), with a compound of the formula (XVII)

$$X'(C_6H_5)_3P—(CH_2)_{n+1}—D \qquad (XVII)$$

wherein n is as defined in formula (I), X' is halo, and D is CN, SCN, or $QR_4$ as defined in formula (I) to provide a compound of formula (I) wherein Y—Z is —CH=CH—; or

(e) reacting a compound of formula (I) wherein D is CN with an alkali metal azide and ammonium chloride, or with tetramethylquanidinium azide to provide a compound of formula (I) wherein D is 5-tetrazolyl; or

(f) reacting a compound of formula IV

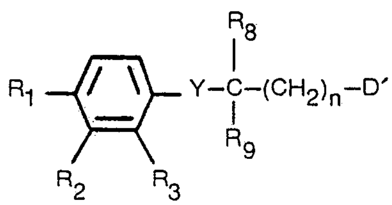

IV

wherein $R_1$, $R_2$, $R_3$, $R_9$, $R_8$, and n are as defined in formula (I) Y is O or S, and D' is halo, with an alkali metal cyanide or an alkali metal thiocyanate to provide a compound of formula (I) wherein D is CN or SCN; or

(g) reacting a compound of formula IV wherein D' is halo with 5-mercaptotetrazole to provide a compound of formula (I) wherein D is thiotetrazole; or

(h) oxidizing a compound of formula (I) wherein Y or Z is S or SO to provide the corresponding compound of formula (I) wherein Y or Z is SO or $SO_2$; and

(i) optionally salifying the resulting compound of formula (I). .

2. The process of claim 1 when used to prepare a compound of formula (I) wherein D is $QR_4$.

3. The process of claim 1 when used to prepare
5-(2-propyl-3,4-dichlorophenoxy)pentanoic acid or a pharmaceutically acceptable salt thereof, or
4-(2-propyl-3,4-dichlorobenzyloxy)butanoic acid or a pharmaceutically acceptable salt thereof, or
6-(2-propyl-3,4-dichlorophenyl)hexanoic acid or a pharmaceutically acceptable salt thereof, or
5-[4-(2-propyl-3,4-dichlorophenoxy)butyl]tetrazole or a pharmaceutically acceptable salt thereof, or
5-[3-(2-propyl-3,4-dichlorobenzyloxy)propyl]tetrazole or a pharmaceutically acceptable salt thereof, or
5-[5-(2-propyl-3,4-dichlorophenyl)pentyl]tetrazole or a pharmaceutically acceptable salt thereof, or
5-[3-(2-propyl-3,4-dichlorophenoxy)propylthio]-tetrazole or a pharmaceutically acceptable salt thereof, or
5-[2-(2-propyl-3,4-dichlorobenzyloxy)ethylthio]-tetrazole or a pharmaceutically acceptable salt thereof, or
5-[4-(2-propyl-3,4-dichlorophenyl)butylthio]-tetrazole or a pharmaceutically acceptable salt thereof.

4. A compound of formula (I) whenever prepared by the process of claim 1.

**Patentansprüche für die Vertragsstaaten: BE CH DE FR GB IT LI LU NL SE**

1. Verbindung der Formel (I)

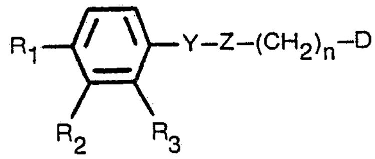

I

oder pharmazeutisch annehmbare Salze hiervon, worin

$R_1$ für

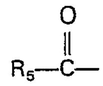 oder Halogen steht,

$R_2$ Halogen oder Hydroxy ist,

$R_3$ für $C_1$—$C_{12}$-Alkyl, hydroxysubstituiertes $C_1$—$C_{12}$-Alkyl oder $C_2$—$C_6$-Alkenyl steht,

Y für —O—,

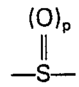 oder —$CR_6R_7$— steht,

Z für —O—,

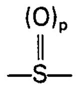 oder —$CR_8R_9$— steht,

oder Y und Z zusammengenommen —CH=CH— sind,

n für 1 bis 10 steht,

D für CN, SCN oder $QR_4$ steht,

Q für —O—, —NR—,

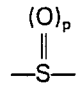 oder eine Bindung steht,

$R_4$ für —$COR_{10}$, Hydroxy, —$NR_{11}R_{12}$, —SC(=NH)$NH_2$ oder

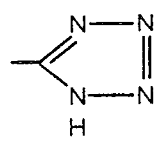

steht, worin

R Wasserstoff oder $C_1$—$C_3$-Alkyl ist,

$R_5$ Wasserstoff, $C_1$—$C_6$-Alkyl, $C_3$—$C_8$-Cycloalkyl oder Phenyl ist, das gegebenenfalls durch Halogen, $C_1$—$C_4$-Alkyl oder $C_1$—$C_4$-Alkoxy substituiert ist,

$R_6$ und $R_7$ unabhängig Wasserstoff, $C_1$—$C_{10}$-Alkyl, Phenyl oder Benzyl sind,

$R_8$ und $R_9$ Wasserstoff bedeuten,

$R_{10}$ Hydroxy, $C_1$—$C_4$-Alkoxy, —NHOH oder —$NR_{11}R_{12}$ ist,

$R_{11}$ und $R_{12}$ jeweils unabhängig Wasserstoff oder $C_1$—$C_3$-Alkyl sind oder zusammen mit dem Stickstoffatom einen Morpholinring oder N-Methylpiperazinring bilden und

p für 0, 1 oder 2 steht,

mit der Maßgabe, daß

a) falls $R_1$ für

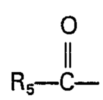 steht, $R_2$ nicht Hydroxy sein kann,

b) falls einer der Substituenten Y und Z für —O— oder

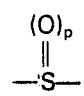 steht,

der andere der Substituenten Y und Z nicht —O— oder

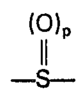 sein kann

und

c) falls $R_4$ für $COR_{10}$, Hydroxy, —$NR_{11}R_{12}$ oder —SC(=NH)$NH_2$ steht, Q nur eine Bindung sein kann.

2. Verbindung der Formel (I), dadurch gekennzeichnet, daß D für $QR_4$ steht.

3. Verbindungen nach Anspruch 1, dadurch gekennzeichnet, daß es sich hierbei um folgende handelt:

5-(2-Propyl-3,4-dichlorphenoxy)pentansäure oder ein pharmazeutisch annehmbares Salz hiervon,

4-(2-Propyl-3,4-dichlorbenzyloxy)butansäure oder ein pharmazeutisch annehmbares Salz hiervon,

6-(2-Propyl-3,4-dichlorphenyl)hexansäure oder ein pharmazeutisch annehmbares Salz hiervon,

5-[4-(2-Propyl-3,4-dichlorphenoxy)butyl]-tetrazol oder ein pharmazeutisch annehmbares Salz hiervon,

5-[3-(2-Propyl-3,4-dichlorbenzyloxy)propyl]-tetrazol oder ein pharmazeutisch annehmbares Salz hiervon,

5-[5-(2-Propyl-3,4-dichlorphenyl)pentyl]-tetrazol oder ein pharmazeutisch annehmbares Salz hiervon,

5-[3-(2-Propyl-3,4-dichlorphenoxy)propylthio]-tetrazol oder ein pharmazeutisch annehmbares Salz hiervon,

5-[2-(2-Propyl-3,4-dichlorbenzyloxy)ethylthio]-tetrazol oder ein pharmazeutisch annehmbares Salz hiervon, oder

5-[4-(2-Propyl-3,4-dichlorphenyl)butylthio]-tetrazol oder ein pharmazeutisch annehmbares Salz hiervon.

4. Pharmazeutische Formulierung, dadurch gekennzeichnet, daß sie eine Verbindung der Formel (I) gemäß irgendeinem der Ansprüche 1 bis 3, sofern D für $QR_4$ steht, als Wirkstoff in Kombination mit einem oder mehreren pharmazeutisch annehmbaren Trägern oder Verdünnungsmitteln hierfür enthält.

5. Verfahren zur Herstellung einer Verbindung der Formel (I), dadurch gekennzeichnet, daß man

(a) eine Verbindung der Formel (II)

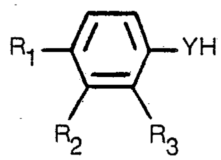

(II)

worin $R_1$, $R_2$ und $R_3$ wie bei der Formel (I) definiert sind und Y für S oder O steht, mit einer Verbindung der Formel (III)

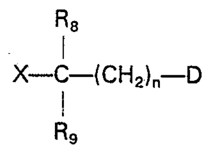

(III)

worin $R_8$, $R_9$ und n wie bei der Formel (I) definiert sind, X eine Abgangsgruppe ist und D für CN, SCN oder $QR_4$ wie bei der Definition der Formel (I) steht, umsetzt und so Verbindungen der Formel (I) bildet, worin Y für S oder O steht, oder

(b) eine Verbindung der Formel

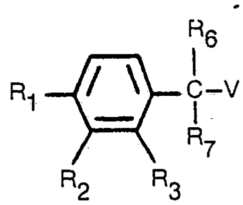

worin $R_1$, $R_2$, $R_3$, $R_6$ und $R_7$ wie bei der Formel (I) definiert sind, mit einer Verbindung der Formel

$$W—(CH_2)_n—D$$

worin n wie bei der Formel (I) definiert ist, D für CN, SCN oder $QR_4$ mit der bei der Formel (I) angegebenen Definition steht und einer der Substituenten V oder W eine Abgangsgruppe ist und der andere für —ZH steht, worin Z für O oder S steht, umsetzt und so eine Verbindung der Formel (I) bildet, worin Z für O oder S steht, oder

(c) eine Verbindung der Formel (XIII)

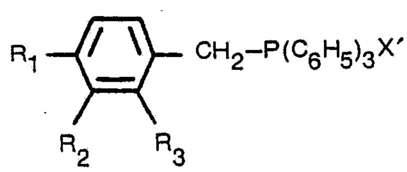

XIII

worin $R_1$, $R_2$ und $R_3$ wie bei der Formel (I) definiert sind und X′ Halogen ist, mit einer Verbindung der Formel (XIV)

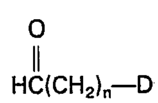

(XIV)

worin n wie bei der Formel (I) definiert ist und D für CN, SCN oder $QR_4$ gemäß Definition bei der Formel (I) steht, umsetzt und so eine Verbindung der Formel (I) bildet, worin Y—Z für —CH=CH— steht, oder

(d) eine Verbindung der Formel (XVI)

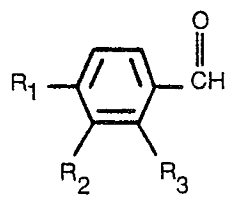

XVI

worin $R_1$, $R_2$ und $R_3$ wie bei der Formel (I) definiert sind, mit einer Verbindung der Formel (XVII)

$$X'(C_6H_5)_3P—(CH_2)_{n+1}—D \qquad (XVII)$$

worin n wie bei der Formel (I) definiert ist, X' Halogen ist und D für CN, SCN oder $QR_4$ gemäß Definition in Formel (I) steht, umsetzt und so eine Verbindung der Formel (I) bildet, worin Y—Z für —CH=CH— steht, oder

(e) eine Verbindung der Formel (I), worin D für CN steht, mit einem Alkalimetallazid und Ammoniumchlorid oder mit Tetramethylguanidiniumazid umsetzt und so eine Verbindung der Formel (I) bildet, worin D für 5-Tetrazolyl steht, oder

(f) eine Verbindung der Formel (IV)

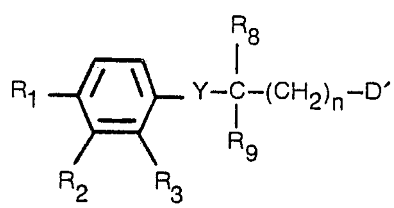

IV

worin $R_1$, $R_2$, $R_3$, $R_8$, $R_9$ und n wie bei der Formel (I) definiert sind, Y für O oder S steht und D' Halogen ist, mit einem Alkalimetallcyanid oder einem Alkalimetallthiocyanat umsetzt und so eine Verbindung der Formel (I) bildet, worin D für CN oder SCN steht, oder

(g) eine Verbindung der Formel (IV), worin D' Halogen ist, mit 5-Mercaptotetrazol umsetzt und so eine Verbindung der Formel (I) bildet, worin D Thiotetrazol ist, oder

(h) eine Verbindung der Formel (I), worin Y oder Z für S oder SO steht, oxidiert und so die entsprechende Verbindung der Formel (I) bildet, worin Y oder Z für SO oder $SO_2$ steht, und

(i) die erhaltene Verbindung der Formel (I) gegebenenfalls in ein Salz überführt.

6. Verwendung einer Verbindung der Formel (I) nach irgendeinem der Ansprüche 1 bis 3, sofern D für $QR_4$ steht, zur Behandlung oder Verhinderung von Zuständen, die durch eine übermäßige Freisetzung von Leukotrienen gekennzeichnet sind, oder zur Behandlung oder Verhinderung einer sofortigen hypersensitiven Reaktion.

7. Verwendung einer Verbindung der Formel (I), worin $R_1$, $R_2$, $R_3$, Y, Z, n und D wie im Anspruch 1 definiert sind, mit der Maßgabe, daß D für $QR_4$ steht und mit der weiteren Maßgabe, daß jeder der Substituenten $R_8$ und $R_9$ unabhängig Wasserstoff, $C_1$—$C_{10}$-Alkyl, Phenyl oder Benzyl ist, zur Herstellung eines Arzneimittels zur Behandlung oder Verhinderung von Zuständen, die durch eine übermäßige Freisetzung von Leukotrienen gekennzeichnet sind, oder zur Behandlung oder Verhinderung einer sofortigen hypersensitiven Reaktion.

**Patentansprüche für den Vertragsstaat: AT**

1. Verfahren zur Herstellung einer Verbindung der Formel (I)

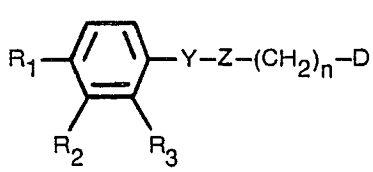

I

oder eines pharmazeutisch annehmbaren Salzes hiervon, worin

$R_1$ für $R_5-\overset{O}{\overset{\|}{C}}-$ oder Halogen steht,

$R_2$ Halogen oder Hydroxy ist,
$R_3$ für $C_1-C_{12}$-Alkyl, hydroxysubstituiertes $C_1-C_{12}$-Alkyl oder $C_2-C_6$-Alkenyl steht,

Y für —O—, $-\overset{(O)_p}{\overset{\|}{S}}-$ oder $-CR_6R_7-$ steht,

Z für —O—, $-\overset{(O)_p}{\overset{\|}{S}}-$ oder $-CR_8R_9-$ steht,

oder Y und Z zusammengenommen —CH=CH— sind,
n für 1 bis 10 steht,
D für CN, SCN oder $QR_4$ steht,
Q für —O—, —NR—, $-\overset{(O)_p}{\overset{\|}{S}}-$ oder eine Bindung steht,

$R_4$ für $-COR_{10}$, Hydroxy, $-NR_{11}R_{12}$, $-SC(=NH)NH_2$ oder

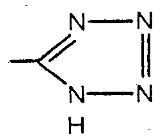

steht, worin
R Wasserstoff oder $C_1-C_3$-Alkyl ist,
$R_5$ Wasserstoff, $C_1-C_6$-Alkyl, $C_3-C_8$-Cycloalkyl oder Phenyl ist, das gegebenenfalls durch Halogen, $C_1-C_4$-Alkyl oder $C_1-C_4$-Alkoxy substituiert ist,
$R_6$ und $R_7$ unabhängig Wasserstoff, $C_1-C_{10}$-Alkyl, Phenyl oder Benzyl sind,
$R_8$ und $R_9$ Wasserstoff bedeuten,
$R_{10}$ Hydroxy, $C_1-C_4$-Alkoxy, —NHOH oder $-NR_{11}R_{12}$ ist,
$R_{11}$ und $R_{12}$ jeweils unabhängig Wasserstoff oder $C_1-C_3$-Alkyl sind oder zusammen mit dem Stickstoffatom einen Morpholinring oder N-Methylpiperazinring bilden und
p für 0, 1 oder 2 steht,
mit der Maßgabe, daß
a) falls $R_1$ für $R_5-\overset{O}{\overset{\|}{C}}-$ steht, $R_2$ nicht Hydroxy sein kann,

b) falls einer der Substituenten Y und Z fur —O— oder $-\overset{(O)_p}{\overset{\|}{S}}-$ steht,

der andere der Substituenten Y und Z nicht —O— oder $-\overset{(O)_p}{\overset{\|}{S}}-$ sein kann

und
c) falls $R_4$ für $COR_{10}$, Hydroxy, $-NR_{11}R_{12}$ oder $-SC(=NH)NH_2$ steht, Q nur eine Bindung sein kann, dadurch gekennzeichnet, daß man

(a) eine Verbindung der Formel (II)

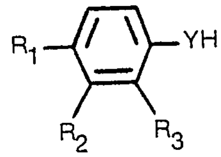

(II)

worin $R_1$, $R_2$ und $R_3$ wie bei der Formel (I) definiert sind und Y für S oder O steht, mit einer Verbindung der Formel (III)

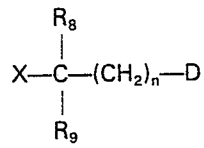

(III)

worin $R_8$, $R_9$ und n wie bei der Formel (I) definiert sind, X eine Abgangsgruppe ist und D für CN, SCN oder $QR_4$ wie bei der Definition der Formel (I) steht, umsetzt und so Verbindungen der Formel (I) bildet, worin Y für S oder O steht, oder

(b) eine Verbindung der Formel

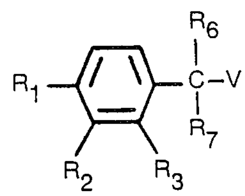

worin $R_1$, $R_2$, $R_3$, $R_6$ und $R_7$ wie bei der Formel (I) definiert sind, mit einer Verbindung der Formel

$$W—(CH_2)_n—D$$

worin n wie bei der Formel (I) definiert ist, D für CN, SCN oder $QR_4$ mit der bei der Formel (I) angegebenen Definition steht und einer der Substituenten V oder W eine Abgangsgruppe ist und der andere für —ZH steht, worin Z für O oder S steht, umsetzt und so eine Verbindung der Formel (I) bildet, worin Z für O oder S steht, oder

(c) eine Verbindung der Formel (XIII)

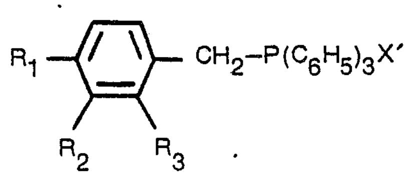

XIII

worin $R_1$, $R_2$ und $R_3$ wie bei der Formel (I) definiert sind und X' Halogen ist, mit einer Verbindung der Formel (XIV)

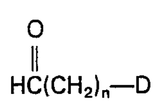

(XIV)

worin n wie bei der Formel (I) definiert ist und D für CN, SCN oder $QR_4$ gemäß Definition bei der Formel (I) steht, umsetzt und so eine Verbindung der Formel (I) bildet, worin Y—Z für —CH=CH— steht, oder

(d) eine Verbindung der Formel (XVI)

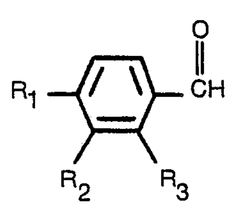

XVI

worin $R_1$, $R_2$ und $R_3$ wie bei der Formel (I) definiert sind, mit einer Verbindung der Formel (XVII)

$X'(C_6H_5)_3P—(CH_2)_{n+1}—D$ (XVII)

worin n wie bei der Formel (I) definiert ist, X' Halogen ist und D für CN, SCN oder $QR_4$ gemäß Definition in Formel (I) steht, umsetzt und so eine Verbindung der Formel (I) bildet, worin Y—Z für —CH=CH— steht, oder

(e) eine Verbindung der Formel (I), worin D für CN steht, mit einem Alkalimetallazid und Ammoniumchlorid oder mit Tetramethylguanidiniumazid umsetzt und so eine Verbindung der Formel (I) bildet, worin D für 5-Tetrazolyl steht, oder

(f) eine Verbindung der Formel (IV)

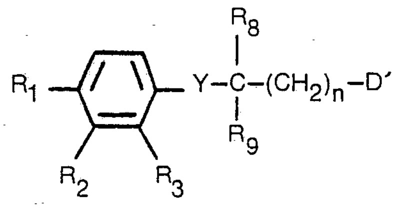

IV

worin $R_1$, $R_2$, $R_3$, $R_8$, $R_9$ und n wie bei der Formel (I) definiert sind, Y für O oder S steht und D' Halogen ist, mit einem Alkalimetallcyanid oder einem Alkalimetallthiocyanat umsetzt und so eine Verbindung der Formel (I) bildet, worin D für CN oder SCN steht, oder

(g) eine Verbindung der Formel (IV), worin D' Halogen ist, mit 5-Mercaptotetrazol umsetzt und so eine Verbindung der Formel (I) bildet, worin D Thiotetrazol ist, oder

(h) eine Verbindung der Formel (I), worin Y oder Z für S oder SO steht, oxidiert und so die entsprechende Verbindung der Formel (I) bildet, worin Y oder Z für SO oder $SO_2$ steht, und

(i) die erhaltene Verbindung der Formel (I) gegebenenfalls in ein Salz überführt.

2. Verfahren nach Anspruch 1, dadurch gekennzeichnet, daß hiernach Verbindungen der Formel (I) hergestellt werden, worin D für $QR_4$ steht.

3. Verfahren nach Anspruch 1, dadurch gekennzeichnet, daß hiernach folgende Verbindungen hergestellt werden:

5-(2-Propyl-3,4-dichlorphenoxy)pentansäure oder ein pharmazeutisch annehmbares Salz hiervon,
4-(2-Propyl-3,4-dichlorbenzyloxy)butansäure oder ein pharmazeutisch annehmbares Salz hiervon,
6-(2-Propyl-3,4-dichlorphenyl)hexansäure oder ein pharmazeutisch annehmbares Salz hiervon,
5-[4-(2-Propyl-3,4-dichlorphenoxy)butyl]-tetrazol oder ein pharmazeutisch annehmbares Salz hiervon,
5-[3-(2-Propyl-3,4-dichlorbenzyloxy)propyl]-tetrazol oder ein pharmazeutisch annehmbares Salz hiervon,
5-[5-(2-Propyl-3,4-dichlorphenyl)pentyl]-tetrazol oder ein pharmazeutisch annehmbares Salz hiervon,
5-[3-(2-Propyl-3,4-dichlorphenoxy)propylthio]-tetrazol oder ein pharmazeutisch annehmbares Salz hiervon,
5-[2-(2-Propyl-3,4-dichlorbenzyloxy)ethylthio]-tetrazol oder ein pharmazeutisch annehmbares Salz hiervon, oder
5-[4-(2-Propyl-3,4-dichlorphenyl)butylthio]-tetrazol oder ein pharmazeutisch annehmbares Salz hiervon.

4. Verbindung der Formel (I), dadurch gekennzeichnet, daß sie nach dem Verfahren von Anspruch 1 hergestellt worden ist.

**Revendications pour les Etats contractants: BE CH DE FR GB IT LI LU NL SE**

1. Composé de formule:

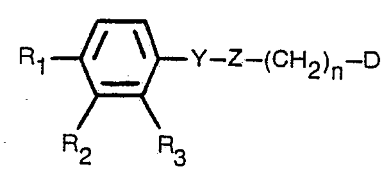

I

ou un de ses sels pharmaceutiquement acceptables, formule dans laquelle:

$R_1$ représente $R_5—C(=O)—$ ou un atome d'halogène;

$R_2$ représente un atome d'halogène ou un groupe hydroxyle;

$R_3$ représente un groupe alkyle en $C_1—C_{12}$, un groupe alkyle en $C_1—C_{12}$ substitué par un groupe hydroxyle ou un groupe alcényle en $C_2—C_6$;

Y représente —O—, $-\overset{(O)_p}{\overset{\|}{S}}-$ ou $-CR_6R_7-$;

Z représente —O—, $-\overset{(O)_p}{\overset{\|}{S}}-$ ou $-CR_8R_9-$

ou, lorsqu'ils sont pris ensemble, —Y—Z représentent —CH=CH—;

n est égal à 1—10;

D représente CN, SCN ou $QR_4$;

Q représente —O—, —NR—, $-\overset{(O)_p}{\overset{\|}{S}}-$ ou une liaison;

$R_4$ représente $-COR_{10}$, un groupe hydroxyle, $-NR_{11}R_{12}$, —SC(=NH)$NH_2$, ou

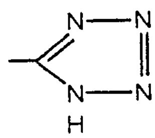

où

R représente un atome d'hydrogène ou un groupe alkyle en $C_1$—$C_3$;

$R_5$ représente un atome d'hydrogène, un groupe alkyle en $C_1$—$C_6$, un groupe cycloalkyle en $C_3$—$C_8$ ou un groupe phényle éventuellement substitué par un atome d'halogène, un groupe alkyle en $C_1$—$C_4$ ou un groupe alcoxy en $C_1$—$C_4$;

$R_6$ et $R_7$ représentent chacun indépendamment l'un de l'autre un atome d'hydrogène, un groupe alkyle en $C_1$—$C_{10}$, un groupe phényle ou un groupe benzyle;

$R_8$ et $R_9$ représentent chacun un atome d'hydrogène;

$R_{10}$ représente un groupe hydroxyle, un groupe alcoxy en $C_1$—$C_4$, —NHOH ou $-NR_{11}R_{12}$;

$R_{11}$ et $R_{12}$ représentent chacun indépendamment l'un de l'autre un atome d'hydrogène, un groupe alkyle en $C_1$—$C_3$ ou, lorsqu'ils sont pris ensemble avec l'atome d'azote, ils forment un noyau morpholine ou un noyau N-méthyl-pipérazine; et

p est égal à 0, 1 ou 2;

avec les réserves suivantes:

a) lorsque $R_1$ représente $R_5-\overset{O}{\overset{\|}{C}}-$, $R_2$ ne peut être un groupe hydroxyle;

b) lorsqu'un des radicaux Y et Z représente —O— ou

$$-\overset{(O)_p}{\overset{\|}{S}}-,$$

l'autre radical Y ou Z ne peut être —O— ou

$$-\overset{(O)_p}{\overset{\|}{S}}-;$$

et

c) lorsque $R_4$ représente $COR_{10}$, un groupe hydroxyle, $-NR_{11}R_{12}$ ou —SC(=NH)$NH_2$, Q ne peut être qu'une liaison.

2. Composé de formule (I) dans laquelle D représente $QR_4$.

3. Composé selon la revendication 1, à savoir

l'acide 5-(2-propyl-3,4-dichlorophénoxy)-pentanoïque ou un de ses sels pharmaceutiquement acceptables, ou

l'acide 4-(2-propyl-3,4-dichlorobenzyloxy)-butanoïque ou un de ses sels pharmaceutiquement acceptables, ou

l'acide 6-(2-propyl-3,4-dichlorophényl)-hexanoïque ou un de ses sels pharmaceutiquement acceptables, ou

le 5-[4-(2-propyl-3,4-dichlorophénoxy)-butyl]-tétrazole ou un de ses sels pharmaceutiquement

acceptables, ou

le 5-[3-(2-propyl-3,4-dichlorobenzyloxy)-propyl]-tétrazole ou un de ses sels pharmaceutiquement acceptables, ou

le 5-[5-(2-propyl-3,4-dichlorophényl)-pentyl]-tétrazole ou un de ses sels pharmaceutiquement acceptables, ou

le 5-[3-(2-propyl-3,4-dichlorophénoxy)-propylthio]-tétrazole ou un de ses sels pharmaceutiquement acceptables, ou

le 5-[2-(2-propyl-3,4-dichlorobenzyloxy)-éthylthio]-tétrazole ou un de ses sels pharmaceutiquement acceptables, ou

le 5-[4-(2-propyl-3,4-dichlorophényl)-butylthio]-tétrazole ou un de ses sels pharmaceutiquement acceptables.

4. Formulation pharmaceutique comprenant un composé de formule (I) comme défini dans l'une quelconque des revendications 1 à 3, pour autant que D représente $QR_4$, en association avec un ou plusieurs diluants ou supports pharmaceutiquement acceptables pour ce composé.

5. Procédé de préparation d'un composé de formule (I), caractérisé en ce qu'il comprend les étapes qui consistent à:

(a) faire réagir un composé de formule (II):

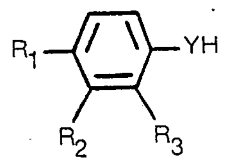

(II)

dans laquelle $R_1$, $R_2$ et $R_3$ ont les définitions données dans la formule (I), tandis que Y représente S ou O, avec un composé de formule (III):

$$X—\underset{R_9}{\overset{R_8}{C}}—(CH_2)_n—D \qquad (III)$$

dans laquelle $R_8$, $R_9$ et n ont les significations définies dans la formule (I), X est un groupe qui s'éloigne et D représente CN, SCN ou $QR_4$ comme défini en formule (I), pour obtenir un composé de formule (I) dans laquelle Y représente S ou O; ou

(b) faire réagir un composé de formule:

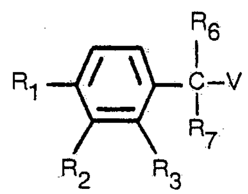

dans laquelle $R_1$, $R_2$, $R_3$, $R_6$ et $R_7$ ont les significations définies dans la formule (I), avec un composé de formule:

$$W—(CH_2)_n—D$$

dans laquelle n a la signification définie en formule (I), D représente CN, SCN ou $QR_4$ comme défini en formule (I), tandis qu'un des radicaux V et W est un groupe qui s'éloigne, tandis que l'autre représente —ZH où Z représente O ou S, pour obtenir un composé de formule (I) dans laquelle Z représente O ou S; ou

(c) faire réagir un composé de formule (XIII):

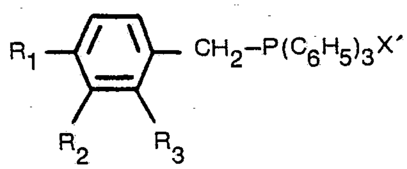

XIII

dans laquelle $R_1$, $R_2$ et $R_3$ ont les significations définies en formule (I) et X' représente un atome d'halogène, avec un composé de formule (XIV):

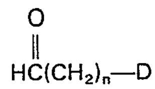

(XIV)

dans laquelle n a la signification définie en formule (I) et D représente CN, SCN ou $QR_4$ comme défini en formule (I) pour obtenir un composé de formule (I) dans laquelle Y—Z représente —CH=CH—; ou

(d) faire réagir un composé de formule (XVI):

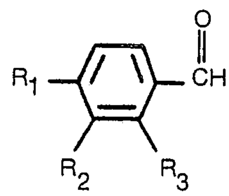

XVI

dans laquelle $R_1$, $R_2$ et $R_3$ ont les significations définies en formule (I), avec un composé de formule (XVII):

$$X'(C_6H_5)_3P—(CH_2)_{n+1}—D \qquad (XVII)$$

dans laquelle n a la signification définie en formule (I), X' représente un atome d'halogène et D représente CN, SCN ou $QR_4$ comme défini en formule (I), pour obtenir un composé de formule (I) dans laquelle Y—Z représente —CH=CH—; ou

(e) faire réagir un composé de formule (I) dans laquelle D représente CN, avec un azide d'un métal alcalin et du chlorure d'ammonium, ou avec un azide de tétraméthyl-guanidinium pour obtenir un composé de formule (I) dans laquelle D représente un groupe 5-tétrazolyle; ou

(f) faire réagir un composé de formule IV:

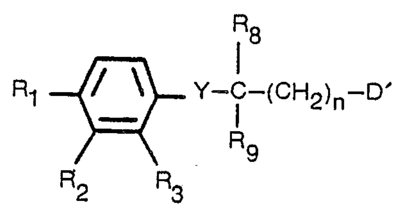

IV

dans laquelle $R_1$, $R_2$, $R_3$, $R_8$, $R_9$ et n ont les significations définies en formule (I), Y représente O ou S et D' représente un atome d'halogène, avec un cyanure d'un métal alcalin ou un thiocyanate d'un métal alcalin, pour obtenir un composé de formule (I) dans laquelle D représente CN ou SCN; ou

(g) faire réagir un composé de formule (IV) dans laquelle D' représente un atome d'halogène, avec du 5-mercaptotétrazole pour obtenir un composé de formule (I) dans laquelle D représente un groupe thiotétrazole; ou

(h) oxyder un composé de formule (I) dans laquelle Y ou Z représente S ou SO, pour obtenir le composé correspondant de formule (I) dans laquelle Y ou Z représente SO ou $SO_2$; et

(i) éventuellement salifier le composé obtenu de formule (I).

6. Composé de formule (I) selon l'une quelconque des revendications 1 à 3, pour autant que D représente $QR_4$, en vue de l'utiliser pour le traitement ou la prévention de n'importe quel état caractérisé par une libération excessive de leucotriènes, ou pour le traitement ou la prévention d'une réaction d'hypersensibilité immédiate.

7. Composé de formule (I) dans laquelle $R_1$, $R_2$, $R_3$, Y, Z, n et D ont les significations définies dans la revendication 1, pour autant que D représente $QR_4$ et pour autant que $R_8$ et $R_9$ représentent chacun indépendamment l'un de l'autre un atome d'hydrogène, un groupe alkyle en $C_1$—$C_{10}$, un groupe phényle ou un groupe benzyle, pour la fabrication d'un médicament pour le traitement et la prévention de n'importe quel état caractérisé par une libération excessive de leucotriènes, ou pour le traitement ou la prévention d'une réaction d'hypersensibilité immédiate.

**Revendications pour l'Etat contractant: AT**

1. Procédé de préparation d'un composé de formule:

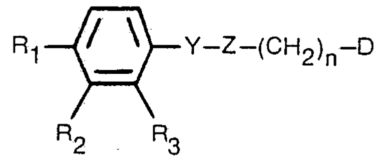

I

ou d'un de ses sels pharmaceutiquement acceptables, formule dans laquelle:

$R_1$ représente 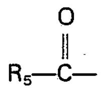 ou un atome d'halogène;

$R_2$ représente un atome d'halogène ou un groupe hydroxyle;

$R_3$ représente un groupe alkyle en $C_1$—$C_{12}$, un groupe alkyle en $C_1$—$C_{12}$ substitué par un groupe hydroxyle ou un groupe alcényle en $C_2$—$C_6$;

Y représente —O—, 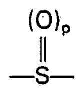 ou —$CR_6R_7$—;

Z représente —O—, 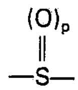 ou —$CR_8R_9$—,

ou, lorsqu'ils sont pris ensemble, —Y—Z représentent —CH=CH—;

n représente 1—10;

D représente CN, SCN ou $QR_4$;

Q représente —O—, —NR—, 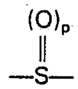 ou une liaison;

$R_4$ représente —$COR_{10}$, un groupe hydroxyle, —$NR_{11}R_{12}$, —SC(=NH)$NH_2$, ou

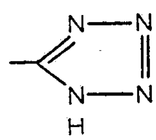

où

R représente un atome d'hydrogène ou un groupe alkyle en $C_1$—$C_3$;

$R_5$ représente un atome d'hydrogène, un groupe alkyle en $C_1$—$C_6$, un groupe cycloalkyle en $C_3$—$C_8$ ou un groupe phényle éventuellement substitué par un atome d'halogène, un groupe alkyle en $C_1$—$C_4$ ou un groupe alcoxy en $C_1$—$C_4$;

$R_6$ et $R_7$ représentent chacun indépendamment l'un de l'autre un atome d'hydrogène, un groupe alkyle en $C_1$—$C_{10}$, un groupe phényle ou un groupe benzyle;

$R_8$ et $R_9$ représentent chacun un atome d'hydrogène;

$R_{10}$ représente un groupe hydroxyle, un groupe alcoxy en $C_1$—$C_4$, —NHOH ou —$NR_{11}R_{12}$;

$R_{11}$ et $R_{12}$ représentent chacun indépendamment l'un de l'autre un atome d'hydrogène, un groupe alkyle en $C_1$—$C_3$ ou, lorsqu'ils sont pris ensemble avec l'atome d'azote, ils forment un noyau morpholine ou un noyau N-méthyl-pipérazine; et

p est égal à 0, 1 ou 2;

avec les réserves suivantes:

a) lorsque $R_1$ représente 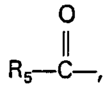, $R_2$ ne peut être un groupe hydroxyle;

b) lorsqu'un des radicaux Y et Z représente —O— ou

$$\overset{(O)_p}{\overset{\|}{—S—}},$$

l'autre radical Y ou Z ne peut être —O— ou

$$\overset{(O)_p}{\overset{\|}{—S—}};$$

et

c) lorsque $R_4$ représente $COR_{10}$, un groupe hydroxyle, $—NR_{11}R_{12}$ ou $—SC(=NH)NH_2$, Q ne peut être qu'une liaison, caractérisé en ce qu'il comprend les étapes qui consistent à:

(a) faire réagir un composé de formule (II):

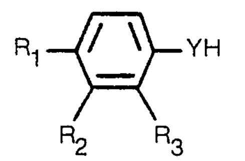

(II)

dans laquelle $R_1$, $R_2$ et $R_3$ ont les significations définies en formule (I) et Y représente S ou O, avec un composé de formule (III):

$$X—\underset{R_9}{\overset{R_8}{\underset{|}{\overset{|}{C}}}}—(CH_2)_n—D \qquad (III)$$

dans laquelle $R_8$, $R_9$ et n ont les significations définies dans en formule (I), X est un groupe qui s'éloigne et D représente CN, SCN ou $QR_4$ comme défini en formule (I), pour obtenir un composé de formule (I) dans laquelle Y représente S ou O; ou

(b) faire réagir un composé de formule:

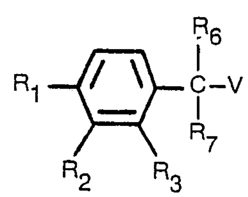

dans laquelle $R_1$, $R_2$, $R_3$, $R_6$ et $R_7$ ont les significations définies en formule (I), avec un composé de formule:

$$W—(CH_2)_n—D$$

dans laquelle n a la signification définie en formule (I), D représente CN, SCN ou $QR_4$ comme défini en formule (I), tandis qu'un des radicaux V et W représente un groupe qui s'éloigne, l'autre représentant —ZH où Z représente O ou S, pour obtenir un composé de formule (I) dans laquelle Z représente O ou S; ou

(c) faire réagir un composé de formule (XIII):

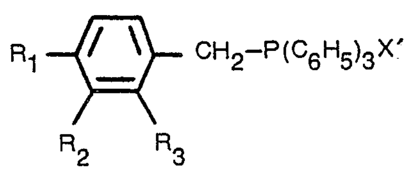

XIII

dans laquelle $R_1$, $R_2$ et $R_3$ ont les significations définies en formule (I) et X' représente un atome d'halogène, avec un composé de formule (XIV):

$$\overset{O}{\overset{\|}{HC}}(CH_2)_n—D \qquad (XIV)$$

dans laquelle n a la signification définie en formule (I) et D représente CN, SCN ou $QR_4$ comme défini en formule (I), pour obtenir un composé de formule (I) dans laquelle Y—Z représente —CH=CH—; ou

(d) faire réagir un composé de formule (XVI):

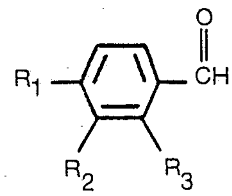

XVI

dans laquelle $R_1$, $R_2$ et $R_3$ ont les significations définies en formule (I), avec un composé de formule (XVII):

$$X'(C_6H_5)_3P—(CH_2)_{n+1}—D \quad (XVII)$$

dans laquelle n a la signification définie en formule (I), X' représente un atome d'halogène et D représente CN, SCN ou $QR_4$ comme défini en formule (I), pour obtenir un composé de formule (I) dans laquelle Y—Z représente —CH=CH—; ou

(e) faire réagir un composé de formule (I) dans laquelle D représente CN, avec un azide d'un métal alcalin et du chlorure d'ammonium, ou avec un azide de tétraméthyl-guanidinium pour obtenir un composé de formule (I) dans laquelle D représente un groupe 5-tétrazolyle; ou

(f) faire réagir un composé de formule (IV):

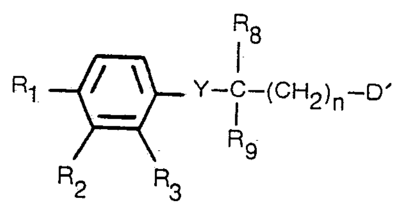

IV

dans laquelle $R_1$, $R_2$, $R_3$, $R_8$, $R_9$ et n ont les significations définies en formule (I), Y représente O ou S et D' représente un atome d'halogène, avec un cyanure d'un métal alcalin ou un thiocyanate d'un métal alcalin pour obtenir un composé de formule (I) dans laquelle D représente CN ou SCN; ou

(g) faire réagir un composé de formule (IV) dans laquelle D' représente un atome d'halogène, avec du 5-mercaptotétrazole pour obtenir un composé de formule (I) dans laquelle D représente un groupe thiotétrazole; ou

(h) oxyder un composé de formule (I) dans laquelle Y ou Z représente S ou SO, pour obtenir le composé correspondant de formule (I) dans laquelle Y ou Z représente SO ou $SO_2$; et

(i) éventuellement salifier le composé obtenu de formule (I).

2. Procédé selon la revendication 1, utilisé pour préparer un composé de formule (I) dans laquelle D représente $QR_4$.

3. Procédé selon la revendication 1, utilisé pour préparer

l'acide 5-(2-propyl-3,4-dichlorophénoxy)-pentanoïque ou un de ses sels pharmaceutiquement acceptables, ou

l'acide 4-(2-propyl-3,4-dichlorobenzyloxy)-butanoïque ou un de ses sels pharmaceutiquement acceptables, ou

l'acide 6-(2-propyl-3,4-dichlorophényl)-hexanoïque ou un de ses sels pharmaceutiquement acceptables, ou

le 5-[4-(2-propyl-3,4-dichlorophénoxy)-butyl]-tétrazole ou un de ses sels pharmaceutiquement acceptables, ou

le 5-[3-(2-propyl-3,4-dichlorobenzyloxy)-propyl]-tétrazole ou un de ses sels pharmaceutiquement acceptables, ou

le 5-[5-(2-propyl-3,4-dichlorophényl)-pentyl]-tétrazole ou un de ses sels pharmaceutiquement acceptables, ou

le 5-[3-(2-propyl-3,4-dichlorophénoxy)-propylthio]-tétrazole ou un de ses sels pharmaceutiquement acceptables, ou

le 5-[2-(2-propyl-3,4-dichlorobenzyloxy)-éthylthio]-tétrazole ou un de ses sels pharmaceutiquement acceptables, ou

le 5-[4-(2-propyl-3,4-dichlorophényl)-butylthio]-tétrazole ou un de ses sels pharmaceutiquement acceptables.

4. Composé de formule (I), préparé par le procédé de la revendication 1.